(19) Europäisches Patentamt
European Patent Office
Office européen des brevets

(11) Veröffentlichungsnummer: **0 269 968**
**A2**

(12) # EUROPÄISCHE PATENTANMELDUNG

(21) Anmeldenummer: **87117201.1**

(22) Anmeldetag: **21.11.87**

(51) Int. Cl.⁴: **C07D 409/12**, C07D 401/12, C07D 403/12, C07D 405/12, C07D 413/12, C07D 417/12, C07D 209/46, C07D 209/48, C07D 217/24, C07D 491/04, A61K 31/40, //(C07D491/04,317:00,221:00),(-C07D491/04,317:00,209:00)

(30) Priorität: **28.11.86 DE 3640641**

(43) Veröffentlichungstag der Anmeldung:
**08.06.88 Patentblatt 88/23**

(84) Benannte Vertragsstaaten:
**AT BE CH DE ES FR GB GR IT LI LU NL SE**

(71) Anmelder: **Dr. Karl Thomae GmbH**
**Postfach 1755**
**D-7950 Biberach 1(DE)**

(72) Erfinder: **Bomhard, Andreas, Dr. Dipl.-Chem.**
**Dinglingerstrasse 9**
**D-7950 Biberach 1(DE)**
Erfinder: **Heider, Joachim, Dr. Dipl.-Chem.**
**Am Hang 3**
**D-7951 Warthausen 1(DE)**
Erfinder: **Psiorz, Manfred, Dr. Dipl.-Chem.**
**Riedlinger Strasse 35**
**D-7950 Biberach 1(DE)**
Erfinder: **Hauel, Norbert, Dr. Dipl.-Chem.**
**Stresemannstrasse 40**
**D-7950 Biberach 1(DE)**
Erfinder: **Narr, Berthod, Dr. Dipl.-Chem.**
**Obere Au 5**
**D-7950 Biberach 1(DE)**
Erfinder: **Noll, Klaus, Dr. Dipl.-Chem.**
**Im Schönblick 3**
**D-7951 Warthausen 1(DE)**
Erfinder: **Lillie, Christian, Dr.**
**Hansi-Niese-Weg 12**
**A-1130 Wien(AT)**
Erfinder: **Kobinger, Walter, Prof. Dr.**
**Belghofergasse 27**
**A-1121 Wien(AT)**
Erfinder: **Diederen, Willi, Dr.**
**Haldenstrasse 1a**
**D-7950 Biberach 1(DE)**

(54) **Neue heteroaromatische Aminderivate, diese Verbindungen enthaltende Arzneimittel und Verfahren zu ihrer Herstellung.**

(57) Die vorliegende Erfindung betrifft neue heteroaromatische Aminderivate der allgemeinen Formel

EP 0 269 968 A2

Xerox Copy Centre

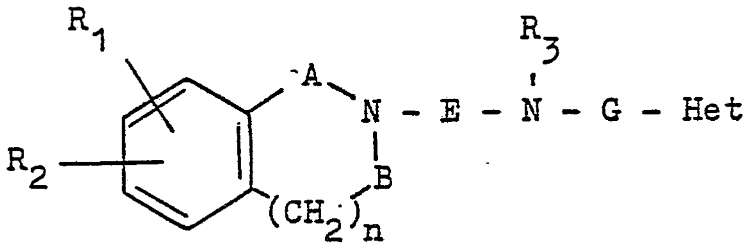

,(I)

in der

n die Zahl 0 oder 1,

A eine Methylen-, Carbonyl-oder Thiocarbonylgruppe,

B eine Methylen-, Carbonyl-oder Thiocarbonylgruppe, wobei nur einer der Reste A oder B eine Thiocarbonylgruppe darstellen kann und in diesem Falle der andere der Reste A oder B eine Methylengruppe darstellen muß,

E eine gegebenenfalls durch eine Alkylgruppe substituierte geradkettige Alkylengruppe,

G eine gegebenenfalls durch eine Alkylgruppe substituierte geradkettige Alkylengruppe,

$R_1$ und $R_2$, die gleich oder verschieden sein konnen, Wasserstoffatome, Alkyl-oder Alkoxygruppen oder $R_1$ und $R_2$ zusammen eine Alkylendioxygruppe,

$R_3$ ein Wasserstoffatom, eine Alkenylgruppe, eine Alkyl-oder Phenylalkylgruppe und

Het einen über ein Kohlenstoff-oder Stickstoffatom gebundenen 5-oder 6-gliedrigen heteroaromatischen Ring, welcher ein Sauerstoff-, Schwefel-oder Stickstoffatom, zwei Stickstoffatome oder ein Stickstoffatom und ein Sauerstoff-oder Schwefelatom enthält, an den zusätzlich über zwei benachbarte Kohlenstoffatome eine 1,3-Propylen-, 1,4-Butylen-oder 1,4-Buta-1,3-dienylengruppe gebunden sein kann, wobei in diesem Falle auch die Bindung über den Kohlenstoffring erfolgen kann, und in denen das Kohlenstoffgerüst der vorstehend erwähnten aromatischen und heteroaromatischen Ringe durch ein Halogenatom, eine Alkyl-, Hydroxy-, Alkoxy-, Phenylalkoxy-, Phenyl-, Dimethoxyphenyl-, Nitro-, Amino-, Acetylamino-, Carbamoylamino-, N-Alkyl-carbamoylamino-, Hydroxymethyl-, Mercapto-, Alkylmercapto-, Alkylsulfinyl-, Alkylsulfonyl-, Alkylsulfonyloxy-, Alkylsulfonylamino-, Alkoxycarbonylmethoxy-, Carboxymethoxy-oder Alkoxymethylgruppe mono-oder disubstituiert oder durch eine Methylendioxy-oder Ethylendioxygruppe substituiert sein kann und gleichzeitig eine gegebenenfalls vorhandene Iminogruppe in den vorstehend erwähnten heteroaromatischen Resten durch eine Alkyl-, Phenylalkyl-oder Phenylgruppe substituiert sein kann, bedeuten, deren N-Oxide, deren Enantiomeren, deren Diastereomeren und deren Säureadditionssalze mit anorganischen oder organischen Säuren, welche wertvolle pharmakologische Eigenschaften aufweisen, inbesondere eine herzfrequenzsenkende und eine positiv inotrope Wirkung.
Die neuen Verbindungen können nach an sich bekannten Verfahren hergestellt werden.

DR. KARL THOMAE GMBH
D-7950 Biberach 1

5/965-Fl/Kp

# Neue heteroaromatische Aminderivate, diese Verbindungen enthaltende Arzneimittel und Verfahren zu ihrer Herstellung

Gegenstand der vorliegenden Erfindung sind neue heteroaromatische Aminderivate der allgemeinen Formel

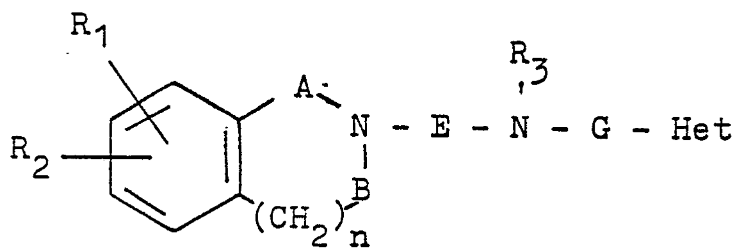 (I)

deren N-Oxide und, falls die Verbindungen ein optisch aktives Kohlenstoffatom enthalten, deren Enantiomeren sowie deren Säureadditionssalze, insbesondere für die pharmazeutische Anwendung deren physiologisch verträgliche Säureadditionssalze mit anorganischen oder organischen Säuren, Verfahren zu ihrer Herstellung und diese Verbindungen enthaltende Arzneimittel.

Die neuen Verbindungen weisen wertvolle pharmakologische Eigenschaften auf, insbesondere neben einer milden blutdrucksenkenden Wirkung und einer positiv inotropen Wirkung eine herzfrequenzsenkende Wirkung und eine herabsetzende Wirkung auf den $O_2$-Bedarf des Herzens.

In der obigen allgemeinen Formel I bedeutet

n die Zahl 0 oder 1,

A eine Methylen-, Carbonyl- oder Thiocarbonylgruppe,

B eine Methylen-, Carbonyl- oder Thiocarbonylgruppe, wobei nur einer der Reste A oder B eine Thiocarbonylgruppe darstellen kann und in diesem Falle der andere der Reste A oder B eine Methylengruppe darstellen muß,

E eine gegebenenfalls durch eine Alkylgruppe mit 1 bis 3 Kohlenstoffatomen substituierte geradkettige Alkylengruppe mit 2 bis 4 Kohlenstoffatomen,

G eine gegebenenfalls durch eine Alkylgruppe mit 1 bis 3 Kohlenstoffatomen substituierte geradkettige Alkylengruppe mit 1 bis 6 Kohlenstoffatomen,

$R_1$ und $R_2$, die gleich oder verschieden sein können, Wasserstoffatome, Alkyl- oder Alkoxygruppen mit jeweils 1 bis 3 Kohlenstoffatomen in jedem Alkylteil oder $R_1$ und $R_2$ zusammen eine Alkylendioxygruppe mit 1 oder 2 Kohlenstoffatomen,

$R_3$ ein Wasserstoffatom, eine Alkenylgruppe mit 3 bis 5 Kohlenstoffatomen, eine Alkyl- oder Phenylalkylgruppe, wobei der Alkylteil jeweils 1 bis 3 Kohlenstoffatome enthalten kann, und

Het einen über ein Kohlenstoff- oder Stickstoffatom gebundenen 5- oder 6-gliedrigen heteroaromatischen Ring, welcher ein Sauerstoff-, Schwefel- oder Stickstoffatom, zwei Stickstoffatome oder ein Stickstoffatom und ein Sauerstoff- oder Schwefelatom enthält, an den zusätzlich über zwei benachbarte Kohlenstoffatome eine 1,3-Propylen-, 1,4-Butylen- oder 1,4-Buta-

1,3-dienylengruppe gebunden sein kann, wobei in diesem Falle auch die Bindung über den Kohlenstoffring erfolgen kann, und in denen das Kohlenstoffgerüst der vorstehend erwähnten aromatischen und heteroaromatischen Ringe durch ein Halogenatom, eine Alkyl-, Hydroxy-, Alkoxy-, Phenylalkoxy-, Phenyl-, Dimethoxyphenyl-, Nitro-, Amino-, Acetylamino-, Carbamoylamino-, N-Alkyl-carbamoylamino-, Hydroxymethyl-, Mercapto-, Alkylmercapto-, Alkylsulfinyl-, Alkylsulfonyl-, Alkylsulfonyloxy-, Alkylsulfonylamino-, Alkoxycarbonylmethoxy-, Carboxymethoxy- oder Alkoxymethylgruppe mono- oder disubstituiert oder durch eine Methylendioxy- oder Ethylendioxygruppe substituiert sein kann und gleichzeitig eine gegebenenfalls vorhandene Iminogruppe in den vorstehend erwähnten heteroaromatischen Resten durch eine Alkyl-, Phenylalkyl- oder Phenylgruppe substituiert sein kann, wobei die vorstehend erwähnten Alkylteile jeweils 1 bis 3 Kohlenstoffatome enthalten können.

Für die bei der Definition der Reste eingangs erwähnten Bedeutungen kommt beispielsweise

für $R_1$ die des Wasserstoffatoms, der Methyl-, Ethyl-, n-Propyl-, Isopropyl-, Methoxy-, Ethoxy-, n-Propoxy- oder Isopropoxygruppe,

für $R_2$ die des Wasserstoffatoms, der Methyl-, Ethyl-, n-Propyl-, Isopropyl-, Methoxy-, Ethoxy-, n-Propoxy- oder Isopropoxygruppe oder zusammen mit $R_1$ die der Methylendioxy- oder Ethylendioxygruppe,

für $R_3$ die des Wasserstoffatoms, der Methyl-, Ethyl-, n-Propyl-, Isopropyl-, Benzyl-, 1-Phenylethyl-, 2-Phenylethyl-, 1-Phenylpropyl-, 1-Methyl-1-phenylethyl-, 3-Phenylpropyl-, Allyl-, n-Buten-(2)-yl- oder n-Penten-(2)-ylgruppe,

für E die der Ethylen-, n-Propylen-, n-Butylen-, 1-Methylethylen-, 2-Ethyl-ethylen-, 1-Propyl-ethylen-, 1-Methyl-n-

propylen-, 2-Methyl-n-propylen-, 1-Ethyl-n-propylen-, 3-Ethyl-n-propylen-, 2-Propyl-n-propylen- oder 2-Methyl-n-butylengruppe,

für G die der Methylen-, Ethyliden-, n-Propyliden-, n-Butyliden-, 2-Methyl-propyliden-, Ethylen-, 1-Methyl-ethylen-, 2-Ethyl-ethylen-, 1-Propyl-ethylen-, 2-Methyl-ethylen-, n-Propylen-, n-Butylen-, n-Pentylen-, n-Hexylen-, 1-Methyl-n-propylen-, 3-Methyl-n-propylen-, 1-Methyl-n-butylen-, 1-Methyl-n-pentylen-, 1-Ethyl-n-propylen-, 2-Ethyl-n-propylen- oder 1-Ethyl-n-butylengruppe und

für Het die der Pyrrolyl-2-, Pyrrolyl-3-, N-Methyl-pyrrolyl-2-, N-Methyl-pyrrolyl-3-, 1,2-Dimethyl-pyrrolyl-3-, 2,5-Dimethyl-pyrrolyl-3-, Furyl-2-, Furyl-3-, 5-Methyl-furyl-2-, 2-Methyl-furyl-3-, 5-Nitro-furyl-2-, 5-Methoxymethyl-furyl-2-, Benzo[b]furyl-2-, Benzo[b]furyl-3-, 7-Methyl-benzo[b]furyl-3-, 2-Methoxy-benzo[b]furyl-3-, 3-Methoxy-benzo[b]furyl-2-, 4-Methoxy-benzo[b]furyl-3-, 5-Methoxy-benzo[b]furyl-3-, 6-Methoxy-benzo[b]furyl-3-, 7-Methoxy-benzo[b]furyl-3-, 5-Methoxy-3-phenyl-benzo[b]furyl-2-, 3-Methyl-5-methoxy-benzo[b]furyl-2-, Thienyl-2-, Thienyl-3-, 5-Methyl-thienyl-2-, 2-Methyl-thienyl-3-, 3-Methyl-thienyl-2-, 2,5-Dimethyl-thienyl-3-, 4,5,6,7-Tetrahydro-benzo[b]thienyl-3-, 4,5,6,7-Tetrahydro-benzo[b]thienyl-2-, 5-Chlor-thienyl-2-, 5-Brom-thienyl-2-, 5-Phenyl-thienyl-2-, 2-Phenyl-thienyl-3-, Benzo[b]thienyl-2-, Benzo[b]thienyl-3-, 2,5-Dimethyl-benzo[b]thienyl-3-, 5-Methyl-benzo[b]thienyl-3-, 6-Methyl-benzo[b]thienyl-3-, 5-Chlor-benzo[b]thienyl-2-, 5-Brom-benzo[b]thienyl-3-, 6-Hydroxy-benzo[b]thienyl-3-, 7-Hydroxy-benzo[b]thienyl-3-, 5-Hydroxy-benzo[b]thienyl-2-, 6-Hydroxy-benzo[b]thienyl-2-, 7-Hydroxy-benzo[b]thienyl-2-, 3-Methoxy-benzo[b]thienyl-2-, 4-Methoxy-benzo[b]thienyl-2-, 5-Methoxy-benzo[b]thienyl-2-, 6-Methoxy-benzo[b]thienyl-2-, 7-Methoxy-benzo[b]thienyl-2-, 2-Methoxy-benzo[b]thienyl-3-, Benzo[b]thienyl-4-, Benzo[b]thienyl-5-, Benzo[b]thienyl-6-,

Benzo[b]thienyl-7-, 4-Methoxy-benzo[b]thienyl-3-, 5-Methoxy-benzo[b]thienyl-3-, 6-Methoxy-benzo[b]thienyl-3-, 7-Methoxy-benzo[b]thienyl-3-, 5,6-Dimethoxy-benzo[b]thienyl-3-, 5,6-Methylendioxy-benzo[b]-thienyl-3-, 6-Ethoxy-benzo[b]thienyl-3-, 6-n-Propoxy-benzo[b]thienyl-3-, 6-Isopropoxy-benzo[b]thienyl-3-, 6-Mercapto-benzo[b]thienyl-3-, 6-Methylmercapto-benzo[b]thienyl-3-, 6-Methylsulfinyl-benzo-[b]thienyl-3-, 6-Methylsulfonyl-benzo[b]thienyl-3-, 6-Methylsulfonyloxy-benzo[b]thienyl-3-, 6-Methoxycarbonylmethoxy-benzo[b]thienyl-3-, 6-Ethoxycarbonylmethoxy-benzo[b]-thienyl-3-, 6-Carboxymethoxy-benzo[b]thienyl-3-, 6-Aminobenzo[b]thienyl-3-, 6-Methylamino-benzo[b]thienyl-3-, 6-Dimethylamino-benzo[b]thienyl-3-, 6-Diethylamino-benzo[b]-thienyl-3-, 6-Acetamino-benzo[b]thienyl-3-, 6-Methylsulfonylamino-benzo[b]thienyl-3-, Pyrazolyl-1-, Pyrazolyl-3-, 3,5-Dimethyl-pyrazolyl-1-, 1,5-Dimethyl-pyrazolyl-3-, Imidazolyl-1-, Imidazolyl-2-, Imidazolyl-4(5)-, 1-Methyl-imidazolyl-4-, 1-Benzyl-imidazolyl-4-, 5-Nitro-2-methyl-imidazolyl-1-, 2-(3,4-Dimethoxy-phenyl)-imidazolyl-4(5)-, Benzo-[d]imidazolyl-1-, 2-Benzyl-benzo[d]imidazolyl-1-, Benzo[d]-imidazolyl-2-, Oxazolyl-4-, Oxazolyl-5-, Isoxazolyl-3-, 3-Methyl-isoxazolyl-5-, 5-Methyl-isoxazolyl-3-, 3,5-Dimethyl-isoxazolyl-4-, 4-Methyl-thiazolyl-5-, Benzo[d]oxazolyl-2-, Benzo[d]isoxazolyl-3-, Benzo[d]thiazolyl-2-, 5-Ethoxy-benzo[d]thiazolyl-2-, Benzo[d]isothiazolyl-3-, Benzo[d]-pyrazolyl-1-, Benzo[d]pyrazolyl-3-, Pyridyl-2-, Pyridyl-3-, Pyridyl-4-, Pyridyl-3-N-oxid-, 6-Methyl-pyridyl-2-, 4-Nitro-pyridyl-2-, 4-Amino-pyridyl-2-, 4-Acetylamino-pyridyl-2-, 4-Carbamoylamino-pyridyl-2-, 4-N-Methyl-carbamoylamino-pyridyl-2-, 2-Chlor-pyridyl-3-, 2-Chlor-pyridyl-4-, 6-Chlor-pyridyl-2-, 6-Hydroxymethyl-pyridyl-2-, Indolyl-2-, Indolyl-3-, 5-Methoxy-indolyl-3-, 5-Methyl-indolyl-3-, 7-Methyl-indolyl-3-, 5-Brom-indolyl-3-, 5-Benzyloxy-indolyl-3-, N-Methyl-indolyl-3-, Chinolyl-2-, Isochinolyl-1-, 2-Methyl-chinolyl-4-, 7-Methyl-chinolyl-2-, 4-Chlor-chinolyl-2-, 6,7-Dimethoxy-chinolyl-4-, 6,7-Dimethoxy-isochinolyl-4- oder 6,7-Dimethoxy-isochinolyl-4-N-oxid-Gruppe in Betracht.

Beispielsweise seien folgende Verbindungen genannt, die unter den Schutzumfang der vorliegenden Erfindung fallen:

2-[N-Methyl-N-(2-(thienyl-2)-ethyl)-3-amino-propyl]-6,7-dimethoxy-1-oxo-1,2,3,4-tetrahydro-isochinolin

2-[N-Methyl-N-(2-(indolyl-3)-ethyl)-3-amino-propyl]-5,6-dimethoxy-phthalimid

2-[N-Methyl-N-(2-(thienyl-2)-ethyl)-3-amino-propyl]-5,6-dimethoxy-1,3-dihydro-1-oxo-isoindol

2-[N-Methyl-N-(3-(pyridyl-4)-propyl)-3-amino-propyl]-6,7-methylendioxy-1-oxo-1,2,3,4-tetrahydro-isochinolin

2-[N-Methyl-N-(3-(pyridyl-4)-propyl)-3-amino-propyl]-5,6-dimethyl-1,3-dihydro-isoindol

2-[N-Methyl-N-(2-(6,7-dimethoxy-isochinolyl-4)-ethyl)-3-amino-propyl]-6,7-dimethoxy-1-oxo-1,2,3,4-tetrahydro-isochinolin

2-[N-Methyl-N-(4-(thienyl-2)-butyl)-3-amino-propyl]-6,7-dimethoxy-1-oxo-1,2,3,4-tetrahydro-isochinolin

2-[N-Methyl-N-(2-(benzo[b]furyl-2)-ethyl)-3-amino-propyl]-6,7-dimethoxy-1-oxo-1,2,3,4-tetrahydro-isochinolin

2-[N-Methyl-N-(2-(benzo[b]thienyl-3)-ethyl)-3-amino-propyl]-6,7-dimethoxy-1-oxo-1,2,3,4-tetrahydro-isochinolin

2-[N-Methyl-N-(2-(thienyl-3)-ethyl)-3-amino-propyl]-6,7-dimethoxy-1-oxo-1,2,3,4-tetrahydro-isochinolin

2-[N-Methyl-N-(2-(indolyl-3)-ethyl)-3-amino-propyl]-6,7-dimethoxy-1-oxo-1,2,3,4-tetrahydro-isochinolin

2-[N-Methyl-N-(3-(indolyl-3)-propyl)-3-amino-propyl]-6,7-dimethoxy-1-oxo-1,2,3,4-tetrahydro-isochinolin

2-[N-Methyl-N-(4-(thienyl-2)-butyl)-3-amino-propyl]-5,6-dimethoxy-phthalimid

2-[N-Methyl-N-(3-(indolyl-3)-propyl)-3-amino-propyl]-5,6-dimethoxy-phthalimid

2-[N-Methyl-N-(2-(thienyl-3)-ethyl)-3-amino-propyl]-5,6-dimethoxy-phthalimid

2-[N-Methyl-N-(2-(benzo[b]thienyl-3)-ethyl)-3-amino-propyl]-5,6-dimethoxy-phthalimid

2-[N-Methyl-N-(2-(thienyl-2)-ethyl)-3-amino-propyl]-5,6-dimethoxy-phthalimid

2-[N-Methyl-N-(3-(pyridyl-3)-propyl)-3-amino-propyl]-6,7-dimethoxy-1-oxo-1,2,3,4-tetrahydro-isochinolin

2-[N-Methyl-N-(3-(pyridyl-3-N-oxid)-propyl)-3-amino-propyl]-6,7-dimethoxy-1-oxo-1,2,3,4-tetrahydro-isochinolin

2-[N-Methyl-N-(2-(indolyl-3)-ethyl)-3-amino-propyl]-5,6-dimethoxy-1,3-dihydro-1-oxo-isoindol

2-[N-Methyl-N-(3-(indolyl-3)-propyl)-3-amino-propyl]-5,6-dimethoxy-1,3-dihydro-1-oxo-isoindol

2-[N-Methyl-N-(2-(thienyl-3)-ethyl)-3-amino-propyl]-5,6-dimethoxy-1,3-dihydro-1-oxo-isoindol

2-[N-Methyl-N-(2-(benzo[b]thienyl-3)-ethyl)-3-amino-propyl]-5,6-dimethoxy-1,3-dihydro-1-oxo-isoindol

2-[N-Methyl-N-(3-(pyridyl-4)-propyl)-3-amino-propyl]-5,6-dimethyl-1,3-dihydro-1-oxo-isoindol

2-[N-Methyl-N-(3-(pyridyl-3)-propyl)-3-amino-propyl]-5,6-dimethoxy-1,3-dihydro-1-oxo-isoindol

2-[N-Methyl-N-((pyridyl-3)-methyl)-3-amino-propyl]-5,6-dimethoxy-1,3-dihydro-1-oxo-isoindol

2-[N-Methyl-N-(2-(6,7-dimethoxy-isochinolyl-4)-ethyl)-3-amino-propyl]-5,6-dimethoxy-1,3-dihydro-1-oxo-isoindol

2-[N-Methyl-N-(2-(6,7-dimethoxy-isochinolyl-4)-ethyl)-3-amino-propyl]-5,6-dimethyl-1,3-dihydro-1-oxo-isoindol

2-[N-Methyl-N-((pyridyl-3)-methyl)-3-amino-propyl]-5,6-dimethyl-1,3-dihydro-1-oxo-isoindol

2-[N-Methyl-N-((pyridyl-3)-methyl)-3-amino-propyl]-6,7-methylendioxy-1-oxo-1,2,3,4-tetrahydro-isochinolin

2-[N-Methyl-N-((pyridyl-3)-methyl)-3-amino-propyl]-5,6-dimethyl-phthalimid

2-[N-Methyl-N-(3-(pyridyl-4)-propyl)-3-amino-propyl]-5,6-dimethyl-phthalimid

2-[N-Methyl-N-(2-(6,7-dimethoxy-isochinolyl-4)-ethyl)-3-amino-propyl]-5,6-dimethyl-phthalimid

2-[N-Methyl-N-(2-(pyridyl-2)-ethyl)-3-amino-propyl]-5,6-dimethoxy-phthalimid

2-[N-Methyl-N-(2-(6,7-dimethoxy-isochinolyl-4)-ethyl)-3-amino-propyl]-5,6-dimethoxy-phthalimid

2-[N-Methyl-N-(2-(6,7-dimethoxy-isochinolyl-4)-ethyl)-3-amino-propyl]-6,7-methylendioxy-1-oxo-1,2,3,4-tetrahydro-isochinolin

2-[N-Methyl-N-(2-(6,7-dimethoxy-isochinolyl-4)-ethyl)-3-amino-propyl]-6,7-methylendioxy-1,2,3,4-tetrahydro-isochinolin

2-[N-Methyl-N-(3-(pyridyl-4)-propyl)-3-amino-propyl]-5,6-dimethoxy-1,3-dihydro-isoindol

2-[N-Methyl-N-(2-(benzo[b]thienyl-3)-ethyl)-3-amino-propyl]-6,7-dimethoxy-1-thioxo-1,2,3,4-tetrahydro-isochinolin

2-[N-Methyl-N-(2-(pyridyl-2)-ethyl)-3-amino-propyl]-6,7-dimethoxy-1-oxo-1,2,3,4-tetrahydro-isochinolin

2-[N-Methyl-N-(3-(pyridyl-4)-propyl)-3-amino-propyl]-6,7-dimethoxy-1-oxo-1,2,3,4-tetrahydro-isochinolin

2-[N-Methyl-N-(2-(pyridyl-3)-ethyl)-3-amino-propyl]-6,7-dimethoxy-1-oxo-1,2,3,4-tetrahydro-isochinolin

2-[N-Methyl-N-(2-(2-methyl-pyridyl-6)-ethyl)-3-amino-propyl]-6,7-dimethoxy-1-oxo-1,2,3,4-tetrahydro-isochinolin

2-[N-Methyl-N-(2-(pyridyl-2)-ethyl)-3-amino-propyl]-6,7-dimethoxy-1,2,3,4-tetrahydro-isochinolin

2-[N-Methyl-N-(3-(pyridyl-3)-propyl)-3-amino-propyl]-6,7-dimethoxy-1,2,3,4-tetrahydro-isochinolin

2-[N-Methyl-N-(3-(pyridyl-4)-propyl)-3-amino-propyl]-6,7-dimethoxy-1,2,3,4-tetrahydro-isochinolin

2-[N-Methyl-N-(2-(6,7-dimethoxy-isochinolyl-4)-ethyl)-3-amino-propyl]-6,7-dimethoxy-1,2,3,4-tetrahydro-isochinolin

2-[N-Methyl-N-(2-(pyridyl-3)-ethyl)-3-amino-propyl]-6,7-dimethoxy-1,2,3,4-tetrahydro-isochinolin

2-[N-Methyl-N-(2-(pyridyl-2)-ethyl)-3-amino-propyl]-6,7-dimethyl-1-oxo-1,2,3,4-tetrahydro-isochinolin

2-[N-Methyl-N-(3-(pyridyl-3)-propyl)-3-amino-propyl]-6,7-dimethyl-1-oxo-1,2,3,4-tetrahydro-isochinolin

2-[N-Methyl-N-(3-(pyridyl-4)-propyl)-3-amino-propyl]-6,7-dimethyl-1-oxo-1,2,3,4-tetrahydro-isochinolin

2-[N-Methyl-N-(3-(pyridyl-3-N-oxido)-propyl)-3-amino-propyl]-6,7-dimethyl-1-oxo-1,2,3,4-tetrahydro-isochinolin

2-[N-Methyl-N-((pyridyl-3)-methyl)-3-amino-propyl]-6,7-dimethyl-1-oxo-1,2,3,4-tetrahydro-isochinolin

2-[N-Methyl-N-(2-(6,7-dimethoxy-isochinolyl-4)-ethyl)-3-amino-propyl]-6,7-dimethyl-1-oxo-1,2,3,4-tetrahydro-isochinolin

2-[N-Methyl-N-(2-(pyridyl-3)-ethyl)-3-amino-propyl]-6,7-dimethyl-1-oxo-1,2,3,4-tetrahydro-isochinolin

2-[N-Methyl-N-(2-(pyridyl-2)-ethyl)-3-amino-propyl]-6,7-dimethyl-1,2,3,4-tetrahydro-isochinolin

2-[N-Methyl-N-(3-(pyridyl-3)-propyl)-3-amino-propyl]-6,7-dimethyl-1,2,3,4-tetrahydro-isochinolin

2-[N-Methyl-N-(2-(pyridyl-3)-ethyl)-3-amino-propyl]-6,7-dimethyl-1,2,3,4-tetrahydro-isochinolin

2-[N-Methyl-N-(2-(pyridyl-2)-ethyl)-3-amino-propyl]-6,7-methylendioxy-1-oxo-1,2,3,4-tetrahydro-isochinolin

2-[N-Methyl-N-(3-(pyridyl-3)-propyl)-3-amino-propyl]-6,7-methylendioxy-1-oxo-1,2,3,4-tetrahydro-isochinolin

2-[N-Methyl-N-(3-(pyridyl-3-N-oxido)-propyl)-3-amino-propyl]-6,7-methylendioxy-1-oxo-1,2,3,4-tetrahydro-isochinolin

2-[N-Methyl-N-(2-(pyridyl-3)-ethyl)-3-amino-propyl]-6,7-methylendioxy-1-oxo-1,2,3,4-tetrahydro-isochinolin

2-[N-Methyl-N-(2-(2-methyl-pyridyl-6)-ethyl)-3-amino-propyl]-6,7-methylendioxy-1-oxo-1,2,3,4-tetrahydro-isochinolin

2-[N-Methyl-N-(2-(pyridyl-2)-ethyl)-3-amino-propyl]-6,7-methylendioxy-1,2,3,4-tetrahydro-isochinolin

2-[N-Methyl-N-(3-(pyridyl-3)-propyl)-3-amino-propyl]-6,7-methylendioxy-1,2,3,4-tetrahydro-isochinolin

2-[N-Methyl-N-(2-(pyridyl-3)-ethyl)-3-amino-propyl]-6,7-methylendioxy-1,2,3,4-tetrahydro-isochinolin

2-[N-Methyl-N-(2-(pyridyl-2)-ethyl)-3-amino-propyl]-6,7-dimethoxy-1,3-dihydro-1-oxo-isoindol

2-[N-Methyl-N-(3-(pyridyl-4)-propyl)-3-amino-propyl]-5,6-dimethoxy-1,3-dihydro-1-oxo-isoindol

2-[N-Methyl-N-(3-(pyridyl-3-N-oxido)-propyl)-3-amino-propyl]-5,6-dimethoxy-1,3-dihydro-1-oxo-isoindol

2-[N-Methyl-N-(2-(pyridyl-3)-ethyl)-3-amino-propyl]-5,6-dimethoxy-1,3-dihydro-1-oxo-isoindol

2-[N-Methyl-N-(2-(2-methyl-pyridyl-6)-ethyl)-3-amino-propyl]-5,6-dimethoxy-1,3-dihydro-1-oxo-isoindol

2-[N-Methyl-N-(2-(6,7-dimethoxy-isochinolyl-4)-ethyl)-3-amino-propyl]-5,6-dimethoxy-1,3-dihydro-isoindol

2-[N-Methyl-N-(2-(pyridyl-3)-ethyl)-3-amino-propyl]-5,6-dimethoxy-1,3-dihydro-isoindol

2-[N-Methyl-N-(2-(pyridyl-2)-ethyl)-3-amino-propyl]-5,6-dimethyl-1,3-dihydro-1-oxo-isoindol

2-[N-Methyl-N-(3-(pyridyl-3)-propyl)-3-amino-propyl]-5,6-dimethyl-1,3-dihydro-1-oxo-isoindol

2-[N-Methyl-N-(2-(pyridyl-3)-ethyl)-3-amino-propyl]-5,6-dimethyl-1,3-dihydro-1-oxo-isoindol

2-[N-Methyl-N-(2-(2-methyl-pyridyl-6)-ethyl)-3-amino-propyl]-5,6-dimethyl-1,3-dihydro-1-oxo-isoindol

2-[N-Methyl-N-(3-(pyridyl-3)-propyl)-3-amino-propyl]-5,6-dimethyl-1,3-dihydro-isoindol

2-[N-Methyl-N-(2-(pyridyl-3)-ethyl)-3-amino-propyl]-5,6-dimethyl-1,3-dihydro-isoindol

2-[N-Methyl-N-(2-(pyridyl-2)-ethyl)-3-amino-propyl]-5,6-methylendioxy-1,3-dihydro-1-oxo-isoindol

2-[N-Methyl-N-(3-(pyridyl-3)-propyl)-3-amino-propyl]-5,6-methylendioxy-1,3-dihydro-1-oxo-isoindol

2-[N-Methyl-N-(3-(pyridyl-4)-propyl)-3-amino-propyl]-5,6-methylendioxy-1,3-dihydro-1-oxo-isoindol

2-[N-Methyl-N-(3-(pyridyl-3-N-oxido)-propyl)-3-amino-propyl]-5,6-methylendioxy-1,3-dihydro-1-oxo-isoindol

2-[N-Methyl-N-(2-(6,7-dimethoxy-isochinolyl-4)-ethyl)-3-amino-propyl]-5,6-methylendioxy-1,3-dihydro-1-oxo-isoindol

2-[N-Methyl-N-(2-(pyridyl-3)-ethyl)-3-amino-propyl]-5,6-methylendioxy-1,3-dihydro-1-oxo-isoindol

2-[N-Methyl-N-(2-(pyridyl-2)-ethyl)-3-amino-propyl]-5,6-methylendioxy-1,3-dihydro-isoindol

2-[N-Methyl-N-(2-(2-methyl-pyridyl-6)-ethyl)-3-amino-propyl]-5,6-methylendioxy-1,3-dihydro-isoindol

2-[N-Methyl-N-(2-(benzo[b]thienyl-2)-ethyl)-3-amino-propyl]-5,6-dimethoxy-1,3-dihydro-1-oxo-isoindol

2-[N-Methyl-N-(2-(4-methoxy-benzo[b]thienyl-3)-ethyl)-3-amino-propyl]-5,6-dimethoxy-1,3-dihydro-1-oxo-isoindol

2-[N-Methyl-N-(2-(5-methoxy-benzo[b]thienyl-3)-ethyl)-3-amino-propyl]-5,6-dimethoxy-1,3-dihydro-1-oxo-isoindol

2-[N-Methyl-N-(2-(6-methoxy-benzo[b]thienyl-3)-ethyl)-3-amino-propyl]-5,6-dimethoxy-1,3-dihydro-1-oxo-isoindol

2-[N-Methyl-N-(2-(5-methyl-benzo[b]thienyl-3)-ethyl)-3-amino-propyl]-5,6-dimethoxy-1,3-dihydro-1-oxo-isoindol

2-[N-Methyl-N-(2-(5-brom-benzo[b]thienyl-3)-ethyl)-3-amino-propyl]-5,6-dimethoxy-1,3-dihydro-1-oxo-isoindol

2-[N-Methyl-N-(2-(6-methylsulfonyloxy-benzo[b]thienyl-3)-ethyl)-3-amino-propyl]-5,6-dimethoxy-1,3-dihydro-1-oxo-isoindol

2-[N-Methyl-N-(2-(6-carboxymethoxy-benzo[b]thienyl-3)-ethyl)-3-amino-propyl]-5,6-dimethoxy-1,3-dihydro-1-oxo-isoindol

2-[N-Methyl-N-(2-(5-methyl-thienyl-2)-ethyl)-3-amino-propyl]-5,6-dimethoxy-1,3-dihydro-1-oxo-isoindol

2-[N-Methyl-N-(2-(5-brom-thienyl-2)-ethyl)-3-amino-propyl]-5,6-dimethoxy-1,3-dihydro-1-oxo-isoindol

2-[N-Methyl-N-(2-(2,5-dimethyl-thienyl-3)-ethyl)-3-amino-propyl]-5,6-dimethoxy-1,3-dihydro-1-oxo-isoindol

2-[N-Methyl-N-(2-(5-methoxy-indolyl-3)-ethyl)-3-amino-propyl]-5,6-dimethoxy-1,3-dihydro-1-oxo-isoindol

2-[N-Methyl-N-(2-(5-benzyloxy-indolyl-3)-ethyl)-3-amino-propyl]-5,6-dimethoxy-1,3-dihydro-1-oxo-isoindol

2-[N-Methyl-N-(2-(5-brom-indolyl-3)-ethyl)-3-amino-propyl]-5,6-dimethoxy-1,3-dihydro-1-oxo-isoindol

2-[N-Methyl-N-(2-(5-methyl-indolyl-3)-ethyl)-3-amino-propyl]-5,6-dimethoxy-1,3-dihydro-1-oxo-isoindol

2-[N-Methyl-N-(2-(7-methyl-indolyl-3)-ethyl)-3-amino-propyl]-5,6-dimethoxy-1,3-dihydro-1-oxo-isoindol

2-[N-Methyl-N-(2-(benzo[b]furyl-2)-ethyl)-3-amino-propyl]-5,6-dimethoxy-1,3-dihydro-1-oxo-isoindol

2-[N-Methyl-N-(2-(benzo[b]furyl-3)-ethyl)-3-amino-propyl]-5,6-dimethoxy-1,3-dihydro-1-oxo-isoindol

2-[N-Methyl-N-(2-(6-methoxy-benzo[b]furyl-3)-ethyl)-3-amino-propyl]-5,6-dimethoxy-1,3-dihydro-1-oxo-isoindol

2-[N-Methyl-N-(2-(7-methoxy-benzo[b]furyl-3)-ethyl)-3-amino-propyl]-5,6-dimethoxy-1,3-dihydro-1-oxo-isoindol

2-[N-Methyl-N-(2-(furyl-2)-ethyl)-3-amino-propyl]-5,6-dimethoxy-1,3-dihydro-1-oxo-isoindol

2-[N-Methyl-N-(2-(furyl-3)-ethyl)-3-amino-propyl]-5,6-dimethoxy-1,3-dihydro-1-oxo-isoindol

2-[N-Methyl-N-(2-(5-methyl-furyl-2)-ethyl)-3-amino-propyl]-5,6-dimethoxy-1,3-dihydro-1-oxo-isoindol

2-[N-Methyl-N-(2-(2,5-dimethyl-furyl-3)-ethyl)-3-amino-propyl]-5,6-dimethoxy-1,3-dihydro-1-oxo-isoindol

2-[N-Methyl-N-(2-(imidazolyl-4(5))-ethyl)-3-amino-propyl]-5,6-dimethoxy-1,3-dihydro-1-oxo-isoindol

2-[N-Methyl-N-(2-(1-methyl-imidazolyl-4)-ethyl)-3-amino-propyl]-5,6-dimethoxy-1,3-dihydro-1-oxo-isoindol

2-[N-Methyl-N-(2-(imidazolyl-1)-ethyl)-3-amino-propyl]-5,6-dimethoxy-1,3-dihydro-1-oxo-isoindol

2-[N-Methyl-N-(2-(1H-benzo[d]imidazolyl-1)-ethyl)-3-amino-propyl]-5,6-dimethoxy-1,3-dihydro-1-oxo-isoindol

2-[N-Methyl-N-(2-(4-methyl-thiazolyl-5)-ethyl)-3-amino-propyl]-5,6-dimethoxy-1,3-dihydro-1-oxo-isoindol

2-[N-Methyl-N-(2-(1-methyl-pyrrolyl-2)-ethyl)-3-amino-propyl]-5,6-dimethoxy-1,3-dihydro-1-oxo-isoindol

2-[N-Methyl-N-(2-(benzo[b]thienyl-2)-ethyl)-3-amino-propyl]-5,6-methylendioxy-1,3-dihydro-1-oxo-isoindol

2-[N-Methyl-N-(2-(4-methoxy-benzo[b]thienyl-3)-ethyl)-3-amino-propyl]-5,6-methylendioxy-1,3-dihydro-1-oxo-isoindol

2-[N-Methyl-N-(2-(5-methoxy-benzo[b]thienyl-3)-ethyl)-3-amino-propyl]-5,6-methylendioxy-1,3-dihydro-1-oxo-isoindol

2-[N-Methyl-N-(2-(6-methoxy-benzo[b]thienyl-3)-ethyl)-3-amino-propyl]-5,6-methylendioxy-1,3-dihydro-1-oxo-isoindol

2-[N-Methyl-N-(2-(5-methyl-benzo[b]thienyl-3)-ethyl)-3-amino-propyl]-5,6-methylendioxy-1,3-dihydro-1-oxo-isoindol

2-[N-Methyl-N-(2-(5-brom-benzo[b]thienyl-3)-ethyl)-3-amino-propyl]-5,6-methylendioxy-1,3-dihydro-1-oxo-isoindol

2-[N-Methyl-N-(2-(6-methylsulfonyloxy-benzo[b]thienyl-3)-ethyl)-3-amino-propyl]-5,6-methylendioxy-1,3-dihydro-1-oxo-isoindol

2-[N-Methyl-N-(2-(6-carboxymethoxy-benzo[b]thienyl-3)-ethyl)-3-amino-propyl]-5,6-methylendioxy-1,3-dihydro-1-oxo-isoindol

2-[N-Methyl-N-(2-(5-methyl-thienyl-2)-ethyl)-3-amino-propyl]-5,6-methylendioxy-1,3-dihydro-1-oxo-isoindol

2-[N-Methyl-N-(2-(5-brom-thienyl-2)-ethyl)-3-amino-propyl]-5,6-methylendioxy-1,3-dihydro-1-oxo-isoindol

2-[N-Methyl-N-(2-(2,5-dimethyl-thienyl-3)-ethyl)-3-amino-propyl]-5,6-methylendioxy-1,3-dihydro-1-oxo-isoindol

2-[N-Methyl-N-(2-(5-methoxy-indolyl-3)-ethyl)-3-amino-propyl]-5,6-methylendioxy-1,3-dihydro-1-oxo-isoindol

2-[N-Methyl-N-(2-(5-benzyloxy-indolyl-3)-ethyl)-3-amino-propyl]-5,6-methylendioxy-1,3-dihydro-1-oxo-isoindol

2-[N-Methyl-N-(2-(5-brom-indolyl-3)-ethyl)-3-amino-propyl]-5,6-methylendioxy-1,3-dihydro-1-oxo-isoindol

2-[N-Methyl-N-(2-(5-methyl-indolyl-3)-ethyl)-3-amino-propyl]-5,6-methylendioxy-1,3-dihydro-1-oxo-isoindol

2-[N-Methyl-N-(2-(7-methyl-indolyl-3)-ethyl)-3-amino-propyl]-5,6-methylendioxy-1,3-dihydro-1-oxo-isoindol

2-[N-Methyl-N-(2-(benzo[b]furyl-2)-ethyl)-3-amino-propyl]-5,6-methylendioxy-1,3-dihydro-1-oxo-isoindol

2-[N-Methyl-N-(2-(benzo[b]furyl-3)-ethyl)-3-amino-propyl]-5,6-methylendioxy-1,3-dihydro-1-oxo-isoindol

2-[N-Methyl-N-(2-(6-methoxy-benzo[b]furyl-3)-ethyl)-3-amino-propyl]-5,6-methylendioxy-1,3-dihydro-1-oxo-isoindol

2-[N-Methyl-N-(2-(7-methoxy-benzo[b]furyl-3)-ethyl)-3-amino-propyl]-5,6-methylendioxy-1,3-dihydro-1-oxo-isoindol

2-[N-Methyl-N-(2-(furyl-2)-ethyl)-3-amino-propyl]-5,6-methylendioxy-1,3-dihydro-1-oxo-isoindol

2-[N-Methyl-N-(2-(furyl-3)-ethyl)-3-amino-propyl]-5,6-methylendioxy-1,3-dihydro-1-oxo-isoindol

2-[N-Methyl-N-(2-(5-methyl-furyl-2)-ethyl)-3-amino-propyl]-5,6-methylendioxy-1,3-dihydro-1-oxo-isoindol

2-[N-Methyl-N-(2-(2,5-dimethyl-furyl-3)-ethyl)-3-amino-propyl]-5,6-methylendioxy-1,3-dihydro-1-oxo-isoindol

2-[N-Methyl-N-(2-(imidazolyl-4(5))-ethyl)-3-amino-propyl]-5,6-methylendioxy-1,3-dihydro-1-oxo-isoindol

2-[N-Methyl-N-(2-(1-methyl-imidazolyl-4)-ethyl)-3-amino-propyl]-5,6-methylendioxy-1,3-dihydro-1-oxo-isoindol

2-[N-Methyl-N-(2-(imidazolyl-1)-ethyl)-3-amino-propyl]-5,6-methylendioxy-1,3-dihydro-1-oxo-isoindol

2-[N-Methyl-N-(2-(1H-benzo[d]imidazolyl-1)-ethyl)-3-amino-propyl]-5,6-methylendioxy-1,3-dihydro-1-oxo-isoindol

2-[N-Methyl-N-(2-(4-methyl-thiazolyl-5)-ethyl)-3-amino-propyl]-5,6-methylendioxy-1,3-dihydro-1-oxo-isoindol

2-[N-Methyl-N-(2-(1-methyl-pyrrolyl-2)-ethyl)-3-amino-propyl]-5,6-methylendioxy-1,3-dihydro-1-oxo-isoindol

2-[N-Methyl-N-(2-(thienyl-2)-ethyl)-3-amino-propyl]-5,6-methylendioxy-1,3-dihydro-1-oxo-isoindol

2-[N-Methyl-N-(2-(thienyl-3)-ethyl)-3-amino-propyl]-5,6-methylendioxy-1,3-dihydro-1-oxo-isoindol

2-[N-Methyl-N-(2-(benzo[b]thienyl-3)-ethyl)-3-amino-propyl]-5,6-methylendioxy-1,3-dihydro-1-oxo-isoindol

2-[N-Methyl-N-(2-(indolyl-3)-ethyl)-3-amino-propyl]-5,6-methylendioxy-1,3-dihydro-1-oxo-isoindol

2-[N-Methyl-N-(2-(benzo[b]thienyl-2)-ethyl)-3-amino-propyl]-5,6-dimethyl-1,3-dihydro-1-oxo-isoindol

2-[N-Methyl-N-(2-(4-methoxy-benzo[b]thienyl-3)-ethyl)-3-amino-propyl]-5,6-dimethyl-1,3-dihydro-1-oxo-isoindol

2-[N-Methyl-N-(2-(5-methoxy-benzo[b]thienyl-3)-ethyl)-3-amino-propyl]-5,6-dimethyl-1,3-dihydro-1-oxo-isoindol

2-[N-Methyl-N-(2-(6-methoxy-benzo[b]thienyl-3)-ethyl)-3-amino-propyl]-5,6-dimethyl-1,3-dihydro-1-oxo-isoindol

2-[N-Methyl-N-(2-(5-methyl-benzo[b]thienyl-3)-ethyl)-3-amino-propyl]-5,6-dimethyl-1,3-dihydro-1-oxo-isoindol

2-[N-Methyl-N-(2-(5-brom-benzo[b]thienyl-3)-ethyl)-3-amino-propyl]-5,6-dimethyl-1,3-dihydro-1-oxo-isoindol

2-[N-Methyl-N-(2-(6-methylsulfonyloxy-benzo[b]thienyl-3)-ethyl)-3-amino-propyl]-5,6-dimethyl-1,3-dihydro-1-oxo-isoindol

2-[N-Methyl-N-(2-(6-carboxymethoxy-benzo[b]thienyl-3)-ethyl)-3-amino-propyl]-5,6-dimethyl-1,3-dihydro-1-oxo-isoindol

2-[N-Methyl-N-(2-(5-methyl-thienyl-2)-ethyl)-3-amino-propyl]-5,6-dimethyl-1,3-dihydro-1-oxo-isoindol

2-[N-Methyl-N-(2-(5-brom-thienyl-2)-ethyl)-3-amino-propyl]-5,6-dimethyl-1,3-dihydro-1-oxo-isoindol

2-[N-Methyl-N-(2-(2,5-dimethyl-thienyl-3)-ethyl)-3-amino-propyl]-5,6-dimethyl-1,3-dihydro-1-oxo-isoindol

2-[N-Methyl-N-(2-(5-methoxy-indolyl-3)-ethyl)-3-amino-propyl]-5,6-dimethyl-1,3-dihydro-1-oxo-isoindol

2-[N-Methyl-N-(2-(5-benzyloxy-indolyl-3)-ethyl)-3-amino-propyl]-5,6-dimethyl-1,3-dihydro-1-oxo-isoindol

2-[N-Methyl-N-(2-(5-brom-indolyl-3)-ethyl)-3-amino-propyl]-5,6-dimethyl-1,3-dihydro-1-oxo-isoindol

2-[N-Methyl-N-(2-(5-methyl-indolyl-3)-ethyl)-3-amino-propyl]-5,6-dimethyl-1,3-dihydro-1-oxo-isoindol

2-[N-Methyl-N-(2-(7-methyl-indolyl-3)-ethyl)-3-amino-propyl]-5,6-dimethyl-1,3-dihydro-1-oxo-isoindol

2-[N-Methyl-N-(2-(benzo[b]furyl-2)-ethyl)-3-amino-propyl]-5,6-dimethyl-1,3-dihydro-1-oxo-isoindol

2-[N-Methyl-N-(2-(benzo[b]furyl-3)-ethyl)-3-amino-propyl]-5,6-dimethyl-1,3-dihydro-1-oxo-isoindol

2-[N-Methyl-N-(2-(6-methoxy-benzo[b]furyl-3)-ethyl)-3-amino-propyl]-5,6-dimethyl-1,3-dihydro-1-oxo-isoindol

2-[N-Methyl-N-(2-(7-methoxy-benzo[b]furyl-3)-ethyl)-3-amino-propyl]-5,6-dimethyl-1,3-dihydro-1-oxo-isoindol

2-[N-Methyl-N-(2-(furyl-2)-ethyl)-3-amino-propyl]-5,6-dimethyl-1,3-dihydro-1-oxo-isoindol

2-[N-Methyl-N-(2-(furyl-3)-ethyl)-3-amino-propyl]-5,6-dimethyl-1,3-dihydro-1-oxo-isoindol

2-[N-Methyl-N-(2-(5-methyl-furyl-2)-ethyl)-3-amino-propyl]-5,6-dimethyl-1,3-dihydro-1-oxo-isoindol

2-[N-Methyl-N-(2-(2,5-dimethyl-furyl-3)-ethyl)-3-amino-propyl]-5,6-dimethyl-1,3-dihydro-1-oxo-isoindol

2-[N-Methyl-N-(2-(imidazolyl-4(5))-ethyl)-3-amino-propyl]-5,6-dimethyl-1,3-dihydro-1-oxo-isoindol

2-[N-Methyl-N-(2-(1-methyl-imidazolyl-4)-ethyl)-3-amino-propyl]-5,6-dimethyl-1,3-dihydro-1-oxo-isoindol

2-[N-Methyl-N-(2-(imidazolyl-1)-ethyl)-3-amino-propyl]-5,6-dimethyl-1,3-dihydro-1-oxo-isoindol

2-[N-Methyl-N-(2-(1H-benzo[d]imidazolyl-1)-ethyl)-3-amino-propyl]-5,6-dimethyl-1,3-dihydro-1-oxo-isoindol

2-[N-Methyl-N-(2-(4-methyl-thiazolyl-5)-ethyl)-3-amino-propyl]-5,6-dimethyl-1,3-dihydro-1-oxo-isoindol

2-[N-Methyl-N-(2-(1-methyl-pyrrolyl-2)-ethyl)-3-amino-propyl]-5,6-dimethyl-1,3-dihydro-1-oxo-isoindol

2-[N-Methyl-N-(2-(thienyl-2)-ethyl)-3-amino-propyl]-5,6-dimethyl-1,3-dihydro-1-oxo-isoindol

2-[N-Methyl-N-(2-(thienyl-3)-ethyl)-3-amino-propyl]-5,6-dimethyl-1,3-dihydro-1-oxo-isoindol

2-[N-Methyl-N-(2-(benzo[b]thienyl-3)-ethyl)-3-amino-propyl]-5,6-dimethyl-1,3-dihydro-1-oxo-isoindol

2-[N-Methyl-N-(2-(indolyl-3)-ethyl)-3-amino-propyl]-5,6-dimethyl-1,3-dihydro-1-oxo-isoindol

2-[N-Methyl-N-(2-(benzo[b]thienyl-2)-ethyl)-3-amino-propyl]-5,6-dimethoxy-1-oxo-1,2,3,4-tetrahydro-isochinolin

2-[N-Methyl-N-(2-(4-methoxy-benzo[b]thienyl-3)-ethyl)-3-amino-propyl]-5,6-dimethoxy-1-oxo-1,2,3,4-tetrahydro-isochinolin

2-[N-Methyl-N-(2-(5-methoxy-benzo[b]thienyl-3)-ethyl)-3-amino-propyl]-5,6-dimethoxy-1-oxo-1,2,3,4-tetrahydro-isochinolin

2-[N-Methyl-N-(2-(6-methoxy-benzo[b]thienyl-3)-ethyl)-3-amino-propyl]-5,6-dimethoxy-1-oxo-1,2,3,4-tetrahydro-isochinolin

2-[N-Methyl-N-(2-(5-methyl-benzo[b]thienyl-3)-ethyl)-3-amino-propyl]-5,6-dimethoxy-1-oxo-1,2,3,4-tetrahydro-isochinolin

2-[N-Methyl-N-(2-(5-brom-benzo[b]thienyl-3)-ethyl)-3-amino-propyl]-5,6-dimethoxy-1-oxo-1,2,3,4-tetrahydro-isochinolin

2-[N-Methyl-N-(2-(6-methylsulfonyloxy-benzo[b]thienyl-3)-3-amino-propyl]-5,6-dimethoxy-1-oxo-1,2,3,4-tetrahydro-isochinolin

2-[N-Methyl-N-(2-(6-carboxymethoxy-benzo[b]thienyl-3)-ethyl)-3-amino-propyl]-5,6-dimethoxy-1-oxo-1,2,3,4-tetrahydro-isochinolin

2-[N-Methyl-N-(2-(5-methyl-thienyl-2)-ethyl)-3-amino-propyl]-5,6-dimethoxy-1-oxo-1,2,3,4-tetrahydro-isochinolin

2-[N-Methyl-N-(2-(5-brom-thienyl-2)-ethyl)-3-amino-propyl]-5,6-dimethoxy-1-oxo-1,2,3,4-tetrahydro-isochinolin

2-[N-Methyl-N-(2-(2,5-dimethyl-thienyl-3)-ethyl)-3-amino-propyl]-5,6-dimethoxy-1-oxo-1,2,3,4-tetrahydro-isochinolin

2-[N-Methyl-N-(2-(5-methoxy-indolyl-3)-ethyl)-3-amino-propyl]-5,6-dimethoxy-1-oxo-1,2,3,4-tetrahydro-isochinolin

2-[N-Methyl-N-(2-(5-benzyloxy-indolyl-3)-ethyl)-3-amino-propyl]-5,6-dimethoxy-1-oxo-1,2,3,4-tetrahydro-isochinolin

2-[N-Methyl-N-(2-(5-brom-indolyl-3)-ethyl)-3-amino-propyl]-5,6-dimethoxy-1-oxo-1,2,3,4-tetrahydro-isochinolin

2-[N-Methyl-N-(2-(5-methyl-indolyl-3)-ethyl)-3-amino-propyl]-5,6-dimethoxy-1-oxo-1,2,3,4-tetrahydro-isochinolin

2-[N-Methyl-N-(2-(7-methyl-indolyl-3)-ethyl)-3-amino-propyl]-5,6-dimethoxy-1-oxo-1,2,3,4-tetrahydro-isochinolin

2-[N-Methyl-N-(2-(benzo[b]furyl-3)-ethyl)-3-amino-propyl]-5,6-dimethoxy-1-oxo-1,2,3,4-tetrahydro-isochinolin

2-[N-Methyl-N-(2-(6-methoxy-benzo[b]furyl-3)-ethyl)-3-amino-propyl]-5,6-dimethoxy-1-oxo-1,2,3,4-tetrahydro-isochinolin

2-[N-Methyl-N-(2-(7-methoxy-benzo[b]furyl-3)-ethyl)-3-amino-propyl]-5,6-dimethoxy-1-oxo-1,2,3,4-tetrahydro-isochinolin

2-[N-Methyl-N-(2-(furyl-2)-ethyl)-3-amino-propyl]-5,6-dimethoxy-1-oxo-1,2,3,4-tetrahydro-isochinolin

2-[N-Methyl-N-(2-(furyl-3)-ethyl)-3-amino-propyl]-5,6-dimethoxy-1-oxo-1,2,3,4-tetrahydro-isochinolin

2-[N-Methyl-N-(2-(5-methyl-furyl-2)-ethyl)-3-amino-propyl]-5,6-dimethoxy-1-oxo-1,2,3,4-tetrahydro-isochinolin

2-[N-Methyl-N-(2-(2,5-dimethyl-furyl-3)-ethyl)-3-amino-propyl]-5,6-dimethoxy-1-oxo-1,2,3,4-tetrahydro-isochinolin

2-[N-Methyl-N-(2-(imidazolyl-4(5))-ethyl)-3-amino-propyl]-5,6-dimethoxy-1-oxo-1,2,3,4-tetrahydro-isochinolin

2-[N-Methyl-N-(2-(1-methyl-imidazolyl-4)-ethyl)-3-amino-propyl]-5,6-dimethoxy-1-oxo-1,2,3,4-tetrahydro-isochinolin

2-[N-Methyl-N-(2-(imidazolyl-1)-ethyl)-3-amino-propyl]-5,6-dimethoxy-1-oxo-1,2,3,4-tetrahydro-isochinolin

2-[N-Methyl-N-(2-(1H-benzo[d]imidazolyl-1)-ethyl)-3-amino-propyl]-5,6-dimethoxy-1-oxo-1,2,3,4-tetrahydro-isochinolin

2-[N-Methyl-N-(2-(4-methyl-thiazolyl-5)-ethyl)-3-amino-propyl]-5,6-dimethoxy-1-oxo-1,2,3,4-tetrahydro-isochinolin

2-[N-Methyl-N-(2-(1-methyl-pyrrolyl-2)-ethyl)-3-amino-propyl]-5,6-dimethoxy-1-oxo-1,2,3,4-tetrahydro-isochinolin

2-[N-Methyl-N-(2-(thienyl-2)-ethyl)-3-amino-propyl]-5,6-methylendioxy-1-oxo-1,2,3,4-tetrahydro-isochinolin

2-[N-Methyl-N-(2-(thienyl-3)-ethyl)-3-amino-propyl]-5,6-methylendioxy-1-oxo-1,2,3,4-tetrahydro-isochinolin

2-[N-Methyl-N-(2-(benzo[b]thienyl-3)-ethyl)-3-amino-propyl]-5,6-methylendioxy-1-oxo-1,2,3,4-tetrahydro-isochinolin

2-[N-Methyl-N-(2-(indolyl-3)-ethyl)-3-amino-propyl]-5,6-methylendioxy-1-oxo-1,2,3,4-tetrahydro-isochinolin

2-[N-Methyl-N-(2-(benzo[b]furyl-2)-ethyl)-3-amino-propyl]-5,6-methylendioxy-1-oxo-1,2,3,4-tetrahydro-isochinolin

2-[N-Methyl-N-(2-(benzo[b]thienyl-2)-ethyl)-3-amino-propyl]-5,6-dimethyl-1-oxo-1,2,3,4-tetrahydro-isochinolin

2-[N-Methyl-N-(2-(4-methoxy-benzo[b]thienyl-3)-ethyl)-3-amino-propyl]-5,6-dimethyl-1-oxo-1,2,3,4-tetrahydro-isochinolin

2-[N-Methyl-N-(2-(5-methoxy-benzo[b]thienyl-3)-ethyl)-3-amino-propyl]-5,6-dimethyl-1-oxo-1,2,3,4-tetrahydro-isochinolin

2-[N-Methyl-N-(2-(6-methoxy-benzo[b]thienyl-3)-ethyl)-3-amino-propyl]-5,6-dimethyl-1-oxo-1,2,3,4-tetrahydro-isochinolin

2-[N-Methyl-N-(2-(5-methyl-benzo[b]thienyl-3)-ethyl)-3-amino-propyl]-5,6-dimethyl-1-oxo-1,2,3,4-tetrahydro-isochinolin

2-[N-Methyl-N-(2-(5-brom-benzo[b]thienyl-3)-ethyl)-3-amino-propyl]-5,6-dimethyl-1-oxo-1,2,3,4-tetrahydro-isochinolin

2-[N-Methyl-N-(2-(6-methylsulfonyloxy-benzo[b]thienyl-3)-ethyl)-3-amino-propyl]-5,6-dimethyl-1-oxo-1,2,3,4-tetrahydro-isochinolin

2-[N-Methyl-N-(2-(6-carboxymethoxy-benzo[b]thienyl-3)-ethyl)-3-amino-propyl]-5,6-dimethyl-1-oxo-1,2,3,4-tetrahydro-isochinolin

2-[N-Methyl-N-(2-(5-methyl-thienyl-2)-ethyl)-3-amino-propyl]-5,6-dimethyl-1-oxo-1,2,3,4-tetrahydro-isochinolin

2-[N-Methyl-N-(2-(5-brom-thienyl-2)-ethyl)-3-amino-propyl]-5,6-dimethyl-1-oxo-1,2,3,4-tetrahydro-isochinolin

2-[N-Methyl-N-(2-(2,5-dimethyl-thienyl-3)-ethyl)-3-amino-propyl]-5,6-dimethyl-1-oxo-1,2,3,4-tetrahydro-isochinolin

2-[N-Methyl-N-(2-(5-methoxy-indolyl-3)-ethyl)-3-amino-propyl]-5,6-dimethyl-1-oxo-1,2,3,4-tetrahydro-isochinolin

2-[N-Methyl-N-(2-(5-benzyloxy-indolyl-3)-ethyl)-3-amino-propyl]-5,6-dimethyl-1-oxo-1,2,3,4-tetrahydro-isochinolin

2-[N-Methyl-N-(2-(5-brom-indolyl-3)-ethyl)-3-amino-propyl]-5,6-dimethyl-1-oxo-1,2,3,4-tetrahydro-isochinolin

2-[N-Methyl-N-(2-(5-methyl-indolyl-3)-ethyl)-3-amino-propyl]-5,6-dimethyl-1-oxo-1,2,3,4-tetrahydro-isochinolin

2-[N-Methyl-N-(2-(7-methyl-indolyl-3)-ethyl)-3-amino-propyl]-5,6-dimethyl-1-oxo-1,2,3,4-tetrahydro-isochinolin

2-[N-Methyl-N-(2-(benzo[b]furyl-3)-ethyl)-3-amino-propyl]-5,6-dimethyl-1-oxo-1,2,3,4-tetrahydro-isochinolin

2-[N-Methyl-N-(2-(6-methoxy-benzo[b]furyl-3)-ethyl)-3-amino-propyl]-5,6-dimethyl-1-oxo-1,2,3,4-tetrahydro-isochinolin

2-[N-Methyl-N-(2-(7-methoxy-benzo[b]furyl-3)-ethyl)-3-amino-propyl]-5,6-dimethyl-1-oxo-1,2,3,4-tetrahydro-isochinolin

2-[N-Methyl-N-(2-(furyl-2)-ethyl)-3-amino-propyl]-5,6-dimethyl-1-oxo-1,2,3,4-tetrahydro-isochinolin

2-[N-Methyl-N-(2-(furyl-3)-ethyl)-3-amino-propyl]-5,6-dimethyl-1-oxo-1,2,3,4-tetrahydro-isochinolin

2-[N-Methyl-N-(2-(5-methyl-furyl-2)-ethyl)-3-amino-propyl]-5,6-dimethyl-1-oxo-1,2,3,4-tetrahydro-isochinolin

2-[N-Methyl-N-(2-(2,5-dimethyl-furyl-3)-ethyl)-3-amino-propyl]-5,6-dimethyl-1-oxo-1,2,3,4-tetrahydro-isochinolin

2-[N-Methyl-N-(2-(imidazolyl-4(5))-ethyl)-3-amino-propyl]-5,6-dimethyl-1-oxo-1,2,3,4-tetrahydro-isochinolin

2-[N-Methyl-N-(2-(1-methyl-imidazolyl-4)-ethyl)-3-amino-propyl]-5,6-dimethyl-1-oxo-1,2,3,4-tetrahydro-isochinolin

2-[N-Methyl-N-(2-(imidazolyl-1)-ethyl)-3-amino-propyl]-5,6-dimethyl-1-oxo-1,2,3,4-tetrahydro-isochinolin

2-[N-Methyl-N-(2-(1H-benzo[d]imidazolyl-1)-ethyl)-3-amino-propyl]-5,6-dimethyl-1-oxo-1,2,3,4-tetrahydro-isochinolin

2-[N-Methyl-N-(2-(4-methyl-thiazolyl-5)-ethyl)-3-amino-propyl]-5,6-dimethyl-1-oxo-1,2,3,4-tetrahydro-isochinolin

2-[N-Methyl-N-(2-(1-methyl-pyrrolyl-2)-ethyl)-3-amino-propyl]-5,6-dimethyl-1-oxo-1,2,3,4-tetrahydro-isochinolin

2-[N-Methyl-N-(2-(thienyl-2)-ethyl)-3-amino-propyl]-5,6-dimethyl-1-oxo-1,2,3,4-tetrahydro-isochinolin

2-[N-Methyl-N-(2-(thienyl-3)-ethyl)-3-amino-propyl]-5,6-dimethyl-1-oxo-1,2,3,4-tetrahydro-isochinolin

2-[N-Methyl-N-(2-(benzo[b]thienyl-3)-ethyl)-3-amino-propyl]-5,6-dimethyl-1-oxo-1,2,3,4-tetrahydro-isochinolin

2-[N-Methyl-N-(2-(indolyl-3)-ethyl)-3-amino-propyl]-5,6-dimethyl-1-oxo-1,2,3,4-tetrahydro-isochinolin

2-[N-Methyl-N-(2-(benzo[b]furyl-2)-ethyl)-3-amino-propyl]-5,6-dimethyl-1-oxo-1,2,3,4-tetrahydro-isochinolin

2-[N-Methyl-N-(3-(thienyl-2)-propyl)-3-amino-propyl]-5,6-dimethoxy-1,3-dihydro-1-oxo-isoindol

2-[N-Methyl-N-(4-(thienyl-2)-butyl)-3-amino-propyl]-5,6-dimethoxy-1,3-dihydro-1-oxo-isoindol

2-[N-Methyl-N-(5-(thienyl-2)-pentyl)-3-amino-propyl]-5,6-dimethoxy-1,3-dihydro-1-oxo-isoindol

2-[N-Methyl-N-(3-(thienyl-3)-propyl)-3-amino-propyl]-5,6-dimethoxy-1,3-dihydro-1-oxo-isoindol

2-[N-Methyl-N-(4-(thienyl-3)-butyl)-3-amino-propyl]-5,6-dimethoxy-1,3-dihydro-1-oxo-isoindol

2-[N-Methyl-N-(5-(thienyl-3)-pentyl)-3-amino-propyl]-5,6-dimethoxy-1,3-dihydro-1-oxo-isoindol

2-[N-Methyl-N-(3-(benzo[b]thienyl-3)-propyl)-3-amino-propyl]-5,6-dimethoxy-1,3-dihydro-1-oxo-isoindol

2-[N-Methyl-N-(4-(benzo[b]thienyl-3)-butyl)-3-amino-propyl]-5,6-dimethoxy-1,3-dihydro-1-oxo-isoindol

2-[N-Methyl-N-(3-(furyl-2)-propyl)-3-amino-propyl]-5,6-dimethoxy-1,3-dihydro-1-oxo-isoindol

2-[N-Methyl-N-(3-(furyl-3)-propyl)-3-amino-propyl]-5,6-dimethoxy-1,3-dihydro-1-oxo-isoindol

2-[N-Methyl-N-(3-(thienyl-2)-propyl)-3-amino-propyl]-5,6-methylendioxy-1,3-dihydro-1-oxo-isoindol

2-[N-Methyl-N-(4-(thienyl-2)-butyl)-3-amino-propyl]-5,6-methylendioxy-1,3-dihydro-1-oxo-isoindol

2-[N-Methyl-N-(5-(thienyl-2)-pentyl)-3-amino-propyl]-5,6-methylendioxy-1,3-dihydro-1-oxo-isoindol

2-[N-Methyl-N-(3-(thienyl-3)-propyl)-3-amino-propyl]-5,6-methylendioxy-1,3-dihydro-1-oxo-isoindol

2-[N-Methyl-N-(4-(thienyl-3)-butyl)-3-amino-propyl]-5,6-methylendioxy-1,3-dihydro-1-oxo-isoindol

2-[N-Methyl-N-(5-(thienyl-3)-pentyl)-3-amino-propyl]-5,6-methylendioxy-1,3-dihydro-1-oxo-isoindol

2-[N-Methyl-N-(3-(benzo[b]thienyl-3)-propyl)-3-amino-propyl]-5,6-methylendioxy-1,3-dihydro-1-oxo-isoindol

2-[N-Methyl-N-(4-(benzo[b]thienyl-3)-butyl)-3-amino-propyl]-5,6-methylendioxy-1,3-dihydro-1-oxo-isoindol

2-[N-Methyl-N-(3-(furyl-2)-propyl)-3-amino-propyl]-5,6-methylendioxy-1,3-dihydro-1-oxo-isoindol

2-[N-Methyl-N-(3-(furyl-3)-propyl)-3-amino-propyl]-5,6-methylendioxy-1,3-dihydro-1-oxo-isoindol

2-[N-Methyl-N-(3-(thienyl-2)-propyl)-3-amino-propyl]-5,6-dimethyl-1,3-dihydro-1-oxo-isoindol

2-[N-Methyl-N-(4-(thienyl-2)-butyl)-3-amino-propyl]-5,6-dimethyl-1,3-dihydro-1-oxo-isoindol

2-[N-Methyl-N-(5-(thienyl-2)-pentyl)-3-amino-propyl]-5,6-dimethyl-1,3-dihydro-1-oxo-isoindol

2-[N-Methyl-N-(3-(thienyl-3)-propyl)-3-amino-propyl]-5,6-dimethyl-1,3-dihydro-1-oxo-isoindol

2-[N-Methyl-N-(4-(thienyl-3)-butyl)-3-amino-propyl]-5,6-dimethyl-1,3-dihydro-1-oxo-isoindol

2-[N-Methyl-N-(5-(thienyl-3)-pentyl)-3-amino-propyl]-5,6-dimethyl-1,3-dihydro-1-oxo-isoindol

2-[N-Methyl-N-(3-(benzo[b]thienyl-3)-propyl)-3-amino-propyl]-5,6-dimethyl-1,3-dihydro-1-oxo-isoindol

2-[N-Methyl-N-(4-(benzo[b]thienyl-3)-butyl)-3-amino-propyl]-5,6-dimethyl-1,3-dihydro-1-oxo-isoindol

2-[N-Methyl-N-(3-(furyl-2)-propyl)-3-amino-propyl]-5,6-dimethyl-1,3-dihydro-1-oxo-isoindol

2-[N-Methyl-N-(3-(furyl-3)-propyl)-3-amino-propyl]-5,6-dimethyl-1,3-dihydro-1-oxo-isoindol

2-[N-Methyl-N-(3-(thienyl-2)-propyl)-3-amino-propyl]-5,6-dimethoxy-1-oxo-1,2,3,4-tetrahydro-isochinolin

2-[N-Methyl-N-(4-(thienyl-2)-butyl)-3-amino-propyl]-5,6-dimethoxy-1-oxo-1,2,3,4-tetrahydro-isochinolin

2-[N-Methyl-N-(5-(thienyl-2)-pentyl)-3-amino-propyl]-5,6-dimethoxy-1-oxo-1,2,3,4-tetrahydro-isochinolin

2-[N-Methyl-N-(3-(thienyl-3)-propyl)-3-amino-propyl]-5,6-dimethoxy-1-oxo-1,2,3,4-tetrahydro-isochinolin

2-[N-Methyl-N-(4-(thienyl-3)-butyl)-3-amino-propyl]-5,6-dimethoxy-1-oxo-1,2,3,4-tetrahydro-isochinolin

2-[N-Methyl-N-(5-(thienyl-3)-pentyl)-3-amino-propyl]-5,6-dimethoxy-1-oxo-1,2,3,4-tetrahydro-isochinolin

2-[N-Methyl-N-(3-(benzo[b]thienyl-3)-propyl)-3-amino-propyl]-5,6-dimethoxy-1-oxo-1,2,3,4-tetrahydro-isochinolin

2-[N-Methyl-N-(4-(benzo[b]thienyl-3)-butyl)-3-amino-propyl]-5,6-dimethoxy-1-oxo-1,2,3,4-tetrahydro-isochinolin

2-[N-Methyl-N-(3-(furyl-2)-propyl)-3-amino-propyl]-5,6-dimethoxy-1-oxo-1,2,3,4-tetrahydro-isochinolin

2-[N-Methyl-N-(3-(furyl-3)-propyl)-3-amino-propyl]-5,6-dimethoxy-1-oxo-1,2,3,4-tetrahydro-isochinolin

2-[N-Methyl-N-(3-(thienyl-2)-propyl)-3-amino-propyl]-5,6-methylendioxy-1-oxo-1,2,3,4-tetrahydro-isochinolin

2-[N-Methyl-N-(4-(thienyl-2)-butyl)-3-amino-propyl]-5,6-methylendioxy-1-oxo-1,2,3,4-tetrahydro-isochinolin

2-[N-Methyl-N-(5-(thienyl-2)-pentyl)-3-amino-propyl]-5,6-methylendioxy-1-oxo-1,2,3,4-tetrahydro-isochinolin

2-[N-Methyl-N-(3-(thienyl-3)-propyl)-3-amino-propyl]-5,6-methylendioxy-1-oxo-1,2,3,4-tetrahydro-isochinolin

2-[N-Methyl-N-(4-(thienyl-3)-butyl)-3-amino-propyl]-5,6-methylendioxy-1-oxo-1,2,3,4-tetrahydro-isochinolin

2-[N-Methyl-N-(5-(thienyl-3)-pentyl)-3-amino-propyl]-5,6-methylendioxy-1-oxo-1,2,3,4-tetrahydro-isochinolin

2-[N-Methyl-N-(3-(benzo[b]thienyl-3)-propyl)-3-amino-propyl]-5,6-methylendioxy-1-oxo-1,2,3,4-tetrahydro-isochinolin

2-[N-Methyl-N-(4-(benzo[b]thienyl-3)-butyl)-3-amino-propyl]-5,6-methylendioxy-1-oxo-1,2,3,4-tetrahydro-isochinolin

2-[N-Methyl-N-(3-(furyl-2)-propyl)-3-amino-propyl]-5,6-methylendioxy-1-oxo-1,2,3,4-tetrahydro-isochinolin

2-[N-Methyl-N-(3-(furyl-3)-propyl)-3-amino-propyl]-5,6-methylendioxy-1-oxo-1,2,3,4-tetrahydro-isochinolin

2-[N-Methyl-N-(3-(thienyl-2)-propyl)-3-amino-propyl]-5,6-dimethyl-1-oxo-1,2,3,4-tetrahydro-isochinolin

2-[N-Methyl-N-(4-(thienyl-2)-butyl)-3-amino-propyl]-5,6-dimethyl-1-oxo-1,2,3,4-tetrahydro-isochinolin

2-[N-Methyl-N-(5-(thienyl-2)-pentyl)-3-amino-propyl]-5,6-dimethyl-1-oxo-1,2,3,4-tetrahydro-isochinolin

2-[N-Methyl-N-(3-(thienyl-3)-propyl)-3-amino-propyl]-5,6-dimethyl-1-oxo-1,2,3,4-tetrahydro-isochinolin

2-[N-Methyl-N-(4-(thienyl-3)-butyl)-3-amino-propyl]-5,6-dimethyl-1-oxo-1,2,3,4-tetrahydro-isochinolin

2-[N-Methyl-N-(5-(thienyl-3)-pentyl)-3-amino-propyl]-5,6-dimethyl-1-oxo-1,2,3,4-tetrahydro-isochinolin

2-[N-Methyl-N-(3-(benzo[b]thienyl-3)-propyl)-3-amino-propyl]-5,6-dimethyl-1-oxo-1,2,3,4-tetrahydro-isochinolin

2-[N-Methyl-N-(4-(benzo[b]thienyl-3)-butyl)-3-amino-propyl]-5,6-dimethyl-1-oxo-1,2,3,4-tetrahydro-isochinolin

2-[N-Methyl-N-(3-(furyl-2)-propyl)-3-amino-propyl]-5,6-dimethyl-1-oxo-1,2,3,4-tetrahydro-isochinolin

2-[N-Methyl-N-(3-(furyl-3)-propyl)-3-amino-propyl]-5,6-dimethyl-1-oxo-1,2,3,4-tetrahydro-isochinolin

2-[N-Methyl-N-(2-(thienyl-2)-ethyl)-3-amino-propyl]-5,6-dimethoxy-1,3-dihydro-1-thioxo-isoindol

2-[N-Methyl-N-(3-(thienyl-2)-propyl)-3-amino-propyl]-5,6-dimethoxy-1,3-dihydro-1-thioxo-isoindol

2-[N-Methyl-N-(4-(thienyl-2)-butyl)-3-amino-propyl]-5,6-dimethoxy-1,3-dihydro-1-thioxo-isoindol

2-[N-Methyl-N-(2-(thienyl-3)-ethyl)-3-amino-propyl]-5,6-dimethoxy-1,3-dihydro-1-thioxo-isoindol

2-[N-Methyl-N-(2-(benzo[b]thienyl-3)-ethyl)-3-amino-propyl]-5,6-dimethoxy-1,3-dihydro-1-thioxo-isoindol

2-[N-Methyl-N-(2-(thienyl-2)-ethyl)-3-amino-propyl]-5,6-methylendioxy-1,3-dihydro-1-thioxo-isoindol

2-[N-Methyl-N-(3-(thienyl-2)-propyl)-3-amino-propyl]-5,6-methylendioxy-1,3-dihydro-1-thioxo-isoindol

2-[N-Methyl-N-(4-(thienyl-2)-butyl)-3-amino-propyl]-5,6-methylendioxy-1,3-dihydro-1-thioxo-isoindol

2-[N-Methyl-N-(2-(thienyl-3)-ethyl)-3-amino-propyl]-5,6-methylendioxy-1,3-dihydro-1-thioxo-isoindol

2-[N-Methyl-N-(2-(benzo[b]thienyl-3)-ethyl)-3-amino-propyl]-5,6-methylendioxy-1,3-dihydro-1-thioxo-isoindol

2-[N-Methyl-N-(2-(thienyl-2)-ethyl)-3-amino-propyl]-5,6-dimethoxy-1,3-dihydro-isoindol

2-[N-Methyl-N-(3-(thienyl-2)-propyl)-3-amino-propyl]-5,6-dimethoxy-1,3-dihydro-isoindol

2-[N-Methyl-N-(4-(thienyl-2)-butyl)-3-amino-propyl]-5,6-dimethoxy-1,3-dihydro-isoindol

2-[N-Methyl-N-(2-(thienyl-3)-ethyl)-3-amino-propyl]-5,6-dimethoxy-1,3-dihydro-isoindol

2-[N-Methyl-N-(2-(benzo[b]thienyl-3)-ethyl)-3-amino-propyl]-5,6-dimethoxy-1,3-dihydro-isoindol

2-[N-Methyl-N-(2-(thienyl-2)-ethyl)-3-amino-propyl]-5,6-dimethyl-1,3-dihydro-1-thioxo-isoindol

2-[N-Methyl-N-(3-(thienyl-2)-propyl)-3-amino-propyl]-5,6-dimethyl-1,3-dihydro-1-thioxo-isoindol

2-[N-Methyl-N-(4-(thienyl-2)-butyl)-3-amino-propyl]-5,6-dimethyl-1,3-dihydro-1-thioxo-isoindol

2-[N-Methyl-N-(2-(thienyl-3)-ethyl)-3-amino-propyl]-5,6-dimethyl-1,3-dihydro-1-thioxo-isoindol

2-[N-Methyl-N-(2-(benzo[b]thienyl-3)-ethyl)-3-amino-propyl]-5,6-dimethyl-1,3-dihydro-1-thioxo-isoindol

2-[N-Methyl-N-(2-(thienyl-2)-ethyl)-3-amino-propyl]-5,6-methylendioxy-1,3-dihydro-isoindol

2-[N-Methyl-N-(3-(thienyl-2)-propyl)-3-amino-propyl]-5,6-methylendioxy-1,3-dihydro-isoindol

2-[N-Methyl-N-(4-(thienyl-2)-butyl)-3-amino-propyl]-5,6-methylendioxy-1,3-dihydro-isoindol

2-[N-Methyl-N-(2-(thienyl-3)-ethyl)-3-amino-propyl]-5,6-methylendioxy-1,3-dihydro-isoindol

2-[N-Methyl-N-(2-(benzo[b]thienyl-3)-ethyl)-3-amino-propyl]-5,6-methylendioxy-1,3-dihydro-isoindol

2-[N-Methyl-N-(2-(thienyl-2)-ethyl)-3-amino-propyl]-5,6-dimethyl-1,3-dihydro-isoindol

2-[N-Methyl-N-(3-(thienyl-2)-propyl)-3-amino-propyl]-5,6-dimethyl-1,3-dihydro-isoindol

2-[N-Methyl-N-(4-(thienyl-2)-butyl)-3-amino-propyl]-5,6-dimethyl-1,3-dihydro-isoindol

2-[N-Methyl-N-(2-(thienyl-3)-ethyl)-3-amino-propyl]-5,6-dimethyl-1,3-dihydro-isoindol

2-[N-Methyl-N-(2-(benzo[b]thienyl-3)-ethyl)-3-amino-propyl]-5,6-dimethyl-1,3-dihydro-isoindol

2-[N-Ethyl-N-(2-(thienyl-2)-ethyl)-3-amino-propyl]-5,6-dimethoxy-1,3-dihydro-1-oxo-isoindol

2-[N-(2-(Thienyl-2)-ethyl)-3-amino-propyl]-5,6-dimethoxy-1,3-dihydro-1-oxo-isoindol

2-[N-(2-(Thienyl-3)-ethyl)-3-amino-propyl]-5,6-dimethoxy-1,3-dihydro-1-oxo-isoindol

2-[N-Methyl-N-(2-(thienyl-2)-ethyl)-3-amino-propyl]-1,3-dihydro-1-oxo-isoindol

2-[N-Methyl-N-(2-(thienyl-3)-ethyl)-3-amino-propyl]-1,3-dihydro-1-oxo-isoindol

2-[N-Methyl-N-(thienyl-2)-methyl)-3-amino-propyl]-1,3-dihydro-1-oxo-isoindol

2-[N-Methyl-N-(2-(thienyl-2)-ethyl)-2-amino-ethyl]-1,3-dihydro-1-oxo-isoindol

2-[N-Methyl-N-(2-(thienyl-2)-ethyl)-4-amino-butyl]-1,3-dihydro-1-oxo-isoindol

Bevorzugte Verbindungen der vorliegenden Erfindung sind jedoch die Verbindungen der allgemeinen Formel

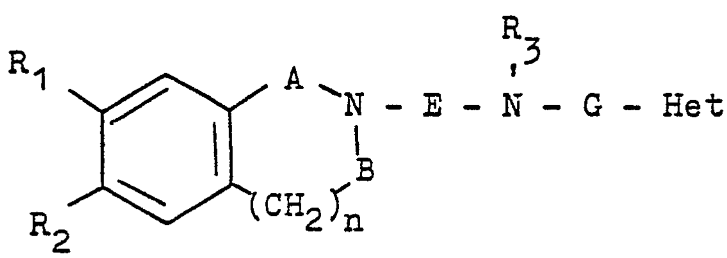

,(Ia)

in der

n die Zahl 0 oder 1,

A eine Methylen- oder Carbonylgruppe oder auch, wenn B eine Methylengruppe darstellt, eine Thiocarbonylgruppe,

B eine Methylen- oder Carbonylgruppe,

E eine n-Propylengruppe,

G eine Methylen-, Äthylen-, n-Propylen-, n-Butylen- oder n-Pentylengruppe,

$R_1$ und $R_2$, die gleich oder verschieden sein können, Wasserstoffatome, Methyl- oder Methoxygruppen oder $R_1$ und $R_2$ zusammen eine Methylendioxygruppe,

$R_3$ ein Wasserstoffatom, eine Methyl- oder Allylgruppe und

Het eine Pyrrolyl-2-, Pyrrolyl-3-, N-Methyl-pyrrolyl-2-, N-Methyl-pyrrolyl-3-, Furyl-2-, Furyl-3-, Benzo[b]furyl-2-, Benzo[b]furyl-3-, 7-Methyl-benzo[b]furyl-3-, 6-Methoxy-benzo-[b]furyl-3-, 5-Methoxy-3-phenyl-benzo[b]furyl-2-, Thienyl-2-, Thienyl-3-, 5-Methyl-thienyl-2-, 2,5-Dimethyl-thienyl-3-, 5-Brom-thienyl-2-, Benzo[b]thienyl-2-, Benzo[b]thienyl-3-, 6-Hydroxy-benzo[b]thienyl-3-, 6-Methoxy-benzo[b]thienyl-3-, 5,6-Dimethoxy-benzo[b]thienyl-3-, 2,5-Dimethyl-benzo[b]-thienyl-3-, 5-Methoxy-benzo[b]thienyl-2-, 6-Methoxy-benzo-[b]thienyl-2-, 6-Methylmercapto-benzo[b]thienyl-3-, 6-Methylsulfinyl-benzo[b]thienyl-3-, 6-Methylsulfonyl-benzo[b]-thienyl-3-, 6-Methylsulfonyloxy-benzo[b]thienyl-3-, 6-Ethoxycarbonylmethoxy-benzo[b]thienyl-3-, 6-Carboxymethoxy-benzo[b]thienyl-3-, 6-Dimethylamino-benzo[b]thienyl-3-, 6-Methylsulfonylamino-benzo[b]thienyl-3-, 6-Acetamino-benzo-[b]thienyl-3-, Benzo[b]thienyl-4-, Pyrazolyl-1-, Pyrazolyl-3-, 1,5-Dimethyl-pyrazolyl-3-, 1-Methyl-imidazolyl-4-, 2-(3,4-Dimethoxy-phenyl)-imidazolyl-4(5)-, Benzo[d]imidazolyl-1-, 2-Benzyl-benzo[d]imidazolyl-1-, Oxazolyl-4-, Oxazolyl-5-, Isoxazolyl-3-, 3-Methyl-isoxazolyl-5-, 4-Methyl-thiazolyl-5-, Pyridyl-2-, Pyridyl-3-, Pyridyl-4-, Pyridyl-3-N-oxid-, 4-Nitro-pyridyl-2-, 4-Amino-pyridyl-2-, 4-Acetylamino-pyridyl-2-, 4-Carbamoylamino-pyridyl-2-, 4-N-Methylcarbamoylamino-pyridyl-2-, Indolyl-2-, Indolyl-3-, 5-Methyl-indolyl-3-, 5-Methoxy-indolyl-3-, N-Methyl-indolyl-3-, 6,7-Dimethoxy-chinolyl-4-, 6,7-Dimethoxy-isochinolyl-4- oder 6,7-Dimethoxy-isochinolyl-4-N-oxid-Gruppe bedeuten, und deren Säureadditionssalze, insbesondere deren physiologisch verträgliche Säureadditionssalze mit anorganischen oder organischen Säuren.

Besonders bevorzugte Verbindungen der obigen allgemeinen Formel Ia sind jedoch diejenigen, in denen

A, B, E, G und n wie vorstehend erwähnt definiert sind,

$R_1$ und $R_2$, die gleich oder verschieden sein können, Methyl- oder Methoxygruppen oder $R_1$ und $R_2$ eine Methylendioxygruppe,

$R_3$ ein Wasserstoffatom oder eine Methylgruppe und

Het eine Thienyl-, Furyl-, Pyridyl-, Pyridyl-3-N-oxid-, Benzo[b]furyl-2-, Benzo[b]furyl-3-, Benzo[b]thienyl-2-, Benzo[b]thienyl-3-, Indolyl-3- oder 6,7-Dimethoxy-isochinolyl-4-gruppe darstellen, und deren Säureadditionssalze, insbesondere deren physiologisch verträgliche Säureadditionssalze mit anorganischen oder organischen Säuren.

Erfindungsgemäß erhält man die neuen Verbindungen nach folgenden Verfahren:

a) Umsetzung einer Verbindung der allgemeinen Formel

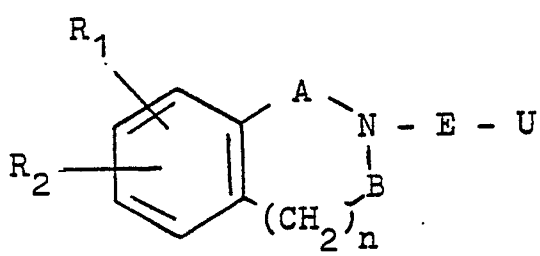

,(II)

mit einer Verbindung der allgemeinen Formel

$$V - G - Het \qquad ,(III)$$

in denen
$R_1$, $R_2$, A, B, E, G, n und Het wie eingangs definiert sind, wobei jedoch A oder B keine Thiocarbonylgruppe darstellen kann,
einer der Reste U oder V die $R_3$'-NH-Gruppe, wobei $R_3$' eine Schutzgruppe für eine Aminogruppe darstellt oder die für $R_3$ eingangs erwähnten Bedeutungen besitzt, und
der andere der Reste U oder V eine nukleophile Austritts-

gruppe wie ein Halogenatom oder eine Sulfonyloxygruppe, z.B. ein Chlor-, Brom- oder Jodatom, die Methansulfonyloxy-, Benzolsulfonyloxy-, p-Toluolsulfonyloxy- oder Ethoxysulfonyloxygruppe, darstellt und gegebenenfalls anschließende Abspaltung eines verwendeten Schutzrestes.

Als Schutzrest für eine Amino- oder Alkylaminogruppe kommt beispielsweise die Acetyl-, Benzoyl-, Ethoxycarbonyl- oder Benzylgruppe in Betracht.

Die Umsetzung wird zweckmäßigerweise in einem Lösungsmittel oder Lösungsmittelgemisch wie Aceton, Diethylether, Methylformamid, Dimethylformamid, Dimethylsulfoxid, Benzol, Toluol, Chlorbenzol, Tetrahydrofuran, Benzol/Tetrahydrofuran, Dioxan oder in einem Überschuß der eingesetzten Verbindungen der allgemeinen Formeln II oder III und gegebenenfalls in Gegenwart eines säurebindenden Mittels, z.B. eines Alkoholats wie Kalium-tert.butylat, eines Alkalihydroxids wie Natrium- oder Kaliumhydroxid, eines Alkalicarbonats wie Kaliumcarbonat, eines Alkaliamids wie Natriumamid, eines Alkalihydrids wie Natriumhydrid, einer tertiären organischen Base wie Triethylamin oder Pyridin, wobei die letzteren gleichzeitig auch als Lösungsmittel dienen können, oder eines Reaktionsbeschleunigers wie Kaliumjodid je nach der Reaktionsfähigkeit des nukleophil austauschbaren Restes zweckmäßigerweise bei Temperaturen zwischen 0 und 150°C, vorzugsweise bei Temperaturen zwischen 50 und 120°C, z.B. bei der Siedetemperatur des verwendeten Lösungsmittels, durchgeführt. Die Umsetzung kann jedoch auch ohne Lösungsmittel durchgeführt werden. Besonders vorteilhaft wird die Umsetzung jedoch in Gegenwart einer tertiären organischen Base oder eines Überschusses des eingesetzten Amins der allgemeinen Formel II oder III durchgeführt.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser,

Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100°C, vorzugsweise bei der Siedetemperatur des Reaktionsgemisches. Die Abspaltung eines Benzylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50°C, vorzugsweise jedoch bei Raumtemperatur, und einem Wasserstoffdruck von 1 bis 7 bar, vorzugsweise jedoch von 3 bis 5 bar.

b) Umsetzung einer Verbindung der allgemeinen Formel

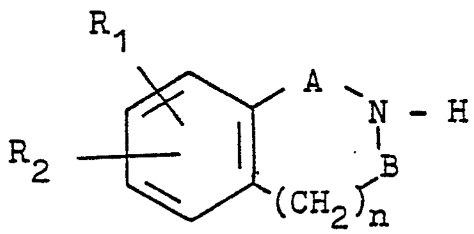

,(IV)

in der

$R_1$, $R_2$, A, B und n wie eingangs definiert sind, wobei jedoch A oder B keine Thiocarbonylgruppe darstellen kann, mit einer Verbindung der allgemeinen Formel

$$Z_1 - E - \overset{R_3'}{\overset{|}{N}} - G - Het \qquad ,(V)$$

in der

E, G und Het wie eingangs definiert sind,

$R_3$' eine Schutzgruppe für eine Aminogruppe darstellt oder die für $R_3$ eingangs erwähnten Bedeutungen besitzt und

$Z_1$ eine nukleophile Austrittsgruppe wie ein Halogenatom oder eine Sulfonyloxygruppe, z.B. ein Chlor-, Brom- oder Jodatom, die Methansulfonyloxy-, p-Toluolsulfonyloxy- oder Ethoxysulfonyloxygruppe, darstellt, und gegebenenfalls anschließende Abspaltung eines verwendeten Schutzrestes.

Als Schutzrest für eine Amino- oder Alkylaminogruppe kommt beispielsweise die Acetyl-, Benzoyl-, Ethoxycarbonyl- oder Benzylgruppe in Betracht.

Die Umsetzung wird zweckmäßigerweise in einem Lösungsmittel oder Lösungsmittelgemisch wie Methylformamid, Dimethylformamid, Dimethylsulfoxid, Benzol, Chlorbenzol, Tetrahydrofuran, Benzol/Tetrahydrofuran oder Dioxan in Gegenwart eines säurebindenden Mittels, z.B. eines Alkoholats wie Kalium-tert.butylat, eines Alkalihydroxids wie Natrium- oder Kaliumhydroxid, eines Alkalicarbonats wie Kaliumcarbonat, eines Alkaliamids wie Natriumamid oder eines Alkalihydrids wie Natriumhydrid zweckmäßigerweise bei Temperaturen zwischen 0 und 150°C, vorzugsweise bei Temperaturen zwischen 0 und 50°C, durchgeführt.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100°C, vorzugsweise bei der Siedetemperatur des Reaktionsgemisches. Die Abspaltung eines Benzylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50°C, vorzugsweise jedoch bei Raumtemperatur, und einem Wasserstoffdruck von 1 bis 7 bar, vorzugsweise jedoch von 3 bis 5 bar.

c) Reduktive Aminierung einer Verbindung der allgemeinen Formel

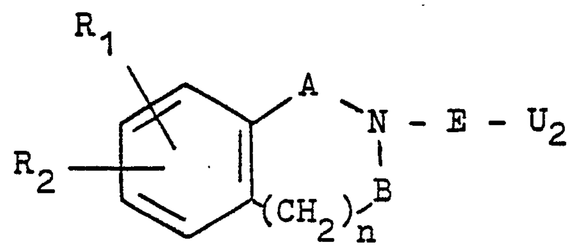

,(VI)

in Gegenwart einer Verbindung der allgemeinen Formel

$$V_2 - G - Het \qquad ,(VII)$$

in denen
$R_1$, $R_2$, A, B, E, G, Het und n wie eingangs definiert sind,
einer der Reste $U_2$ oder $V_2$ eine $R_3$-NH-Gruppe, wobei $R_3$ wie eingangs definiert ist, und
der andere der Reste $U_2$ oder $V_2$ zusammen mit einem Wasserstoffatom des benachbarten Kohlenstoffatoms des Restes G oder E, wobei E und G jeweils wie eingangs definiert sind, ein Sauerstoffatom bedeuten.

Die Reduktion wird in einem geeigneten Lösungsmittel wie Methanol, Ethanol, Diethylether, Tetrahydrofuran, Dioxan, Essigsäureethylester oder Ethanol/Essigsäureethylester mit einem Metallhydrid wie Lithiumaluminiumhydrid, Diboran, Natriumcyanborhydrid oder Boran/Dimethylsulfid, vorzugsweise jedoch mit Natriumborhydrid, oder mit Wasserstoff in Gegenwart eines Hydrierungskatalysators wie Platin, Palladium/-

Kohle oder Raney-Nickel bei einem Wasserstoffdruck von 1 bis 5 bar oder mit Hydrazin in Gegenwart eines Hydrierungskatalysators wie Platin, Palladium/Kohle oder Raney-Nickel, bei Temperaturen zwischen 0 und 50°C, vorzugsweise bei Raumtemperatur, durchgeführt.

Bei der Reduktion mit einem komplexen Metallhydrid wie Lithiumaluminiumhydrid, Diboran oder Boran/Dimethylsulfid, kann eine in den Resten A und/oder B vorhandene Carbonylfunktion zu einer Methylengruppe oder bei der katalytischen Hydrierung eine im Rest $R_3$ vorhandene Doppelbindung mitreduziert werden. Außerdem kann eine in den Resten A oder B vorhandene Thiocarbonylgruppe oder eine in dem Rest Het vorhandene Carbonylfunktion gleichzeitig mitreduziert werden.

d) Reduktion eines Säureamids der allgemeinen Formel

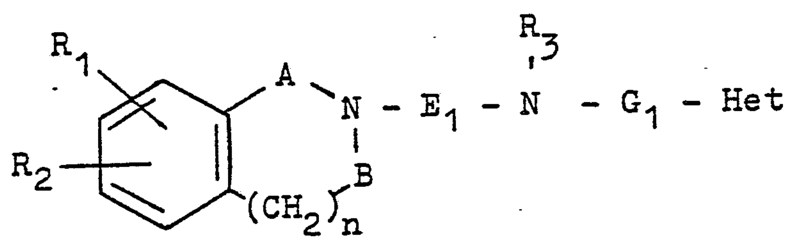

,(VIII)

in der
$R_1$ bis $R_3$, A, B, Het und n wie eingangs definiert sind, einer der Reste $E_1$ oder $G_1$ die für E oder G eingangs erwähnten Bedeutungen aufweist und der andere der Reste $E_1$ oder $G_1$ ebenfalls die für E oder G eingangs erwähnten Bedeutungen besitzt, wobei jedoch eine zu einem Stickstoffatom benachbarte Methylengruppe durch eine Carbonylgruppe ersetzt sein muß.

Die Reduktion wird vorzugsweise in einem geeigneten Lösungsmittel wie Methanol, Ethanol, Diethylether oder Tetrahydrofuran mit einem geeigneten Reduktionsmittel, z.B. mit einem Metallhydrid wie Lithiumaluminiumhydrid, Diboran, Boran/Di-

methylsulfid oder Natriumcyanborhydrid, vorzugsweise jedoch mit Diboran in Tetrahydrofuran, zwischen 0 und 40°C, vorzugsweise jedoch bei Raumtemperatur, durchgeführt.

Bei der Reduktion mit einem komplexen Metallhydrid wie Lithiumaluminiumhydrid, Diboran oder Boran/Dimethylsulfid, kann eine in den Resten A und/oder B vorhandene Carbonylfunktion zu einer Methylengruppe mitreduziert werden. Außerdem kann eine in den Resten A oder B vorhandene Thiocarbonylgruppe oder eine in dem Rest Het vorhandene Carbonylfunktion gleichzeitig mitreduziert werden.

e) Zur Herstellung von Verbindungen der allgemeinen Formel I, in der A oder B eine Thiocarbonylgruppe darstellt:

Umsetzung einer Verbindung der allgemeinen Formel

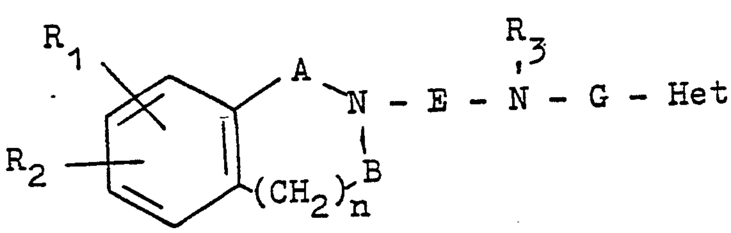

,(IX)

in der
$R_1$ bis $R_3$, A, B, E, G, Het und n wie eingangs definiert sind, wobei jedoch einer der Reste A oder B eine Carbonylgruppe und der andere der Reste eine Methylengruppe darstellen muß, mit einem schwefeleinführenden Mittel.

Die Umsetzung wird mit einem schwefeleinführenden Mittel wie Phosphorpentasulfid oder 2,4-Bis-(4-methoxyphenyl)-1,3-dithia-2,4-diphosphetan-2,4-disulfid zweckmäßigerweise in einem Lösungsmittel wie Toluol oder Xylol bei Temperaturen zwischen 50 und 150°C, z.B. bei der Siedetemperatur des Reaktionsgemisches, durchgeführt.

f) Zur Herstellung von Verbindungen der allgemeinen Formel I, in der A und B jeweils eine $-CH_2$-Gruppe darstellen:

Reduktion einer Verbindung der allgemeinen Formel

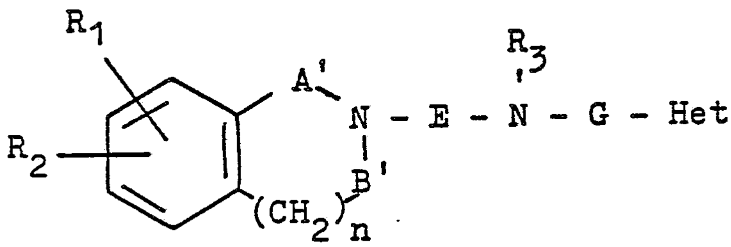

(X)

in der

$R_1$ bis $R_3$, E, G, Het und n wie eingangs definiert sind,
einer der Reste A' oder B' eine Carbonyl- oder Thiocarbonylgruppe und
der andere der Reste A' oder B' eine Methylen-, Carbonyl- oder Thionylcarbonylgruppe darstellen.

Die Reduktion wird vorzugsweise mit einem Metallhydrid wie Lithiumaluminiumhydrid oder Diboran oder mit einem Komplex aus Boran und einem Thioäther, z.B. mit Boran-Dimethylsulfid-Komplex, in einem geeigneten Lösungsmittel wie Diäthyläther oder Tetrahydrofuran bei Temperaturen zwischen 0 und 50°C, vorzugsweise jedoch bei Temperaturen zwischen 10 und 25°C, durchgeführt. Außerdem kann eine in dem Rest Het vorhandene Carbonylfunktion gleichzeitig mitreduziert werden.

g) Zur Herstellung von Verbindungen der allgemeinen Formel I, in der A eine Methylengruppe darstellt:

Reduktion einer Verbindung der allgemeinen Formel

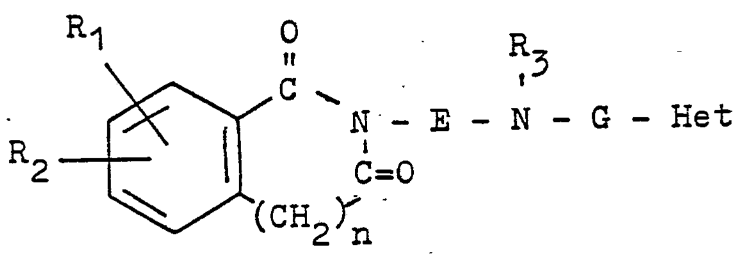

(XI)

in der

$R_1$ bis $R_3$, E, G, Het und n wie eingangs definiert sind.

Die Reduktion wird vorzugsweise in einem geeigneten Lösungsmittel wie Eisessig, Wasser, Äthanol oder Wasser/Eisessig zweckmäßigerweise mit nascierendem Wasserstoff, z.B. in Gegenwart von Zink/Eisessig, Zinn/Salzsäure oder Zinn(II)-chlorid/Salzsäure, oder mit katalytisch angeregtem Wasserstoff bei Temperaturen zwischen 0 und 150°C, vorzugsweise bei Temperaturen zwischen 20 und 125°C, durchgeführt. Hierbei kann eine im Rest $R_3$ vorhandene Doppelbindung oder eine im Rest Het enthaltene Nitrogruppe gleichzeitig mitreduziert werden.

Die erhaltenen Verbindungen der allgemeinen Formel I lassen sich, falls diese ein chirales Zentrum besitzen, mittels üblicher Methoden in ihre Diastereomeren, beispielsweise durch Säulenchromatographie, und in ihre Enantiomeren auftrennen, beispielsweise durch Säulenchromatographie an einer chiralen Phase oder durch Kristallisation mit optisch aktiven Säuren, z.B. mit D- oder L-Monomethylweinsäure, D- oder L-Diacetylweinsäure, D- oder L-Weinsäure, D- oder L-Milchsäure oder D- oder L-Camphersäure.

Die erhaltenen Verbindungen der allgemeinen Formel I lassen sich ferner in ihre Säureadditionssalze, insbesondere für ihre pharmazeutische Anwendung in ihre physiologisch verträglichen Säureadditionssalze mit anorganischen oder organischen Säuren überführen. Als Säuren kommen hierbei beispielsweise Salzsäure, Bromwasserstoffsäure, Schwefelsäure, Phosphorsäure, Essigsäure, Milchsäure, Zitronensäure, Weinsäure, Bernsteinsäure, Maleinsäure oder Fumarsäure in Betracht.

Die als Ausgangsstoffe verwendeten Verbindungen der allgemeinen Formeln II bis XI sind teilweise literaturbekannt bzw. man erhält diese nach an sich bekannten Verfahren.

So erhält man beispielsweise eine als Ausgangsstoff verwendete Verbindung der allgemeinen Formel

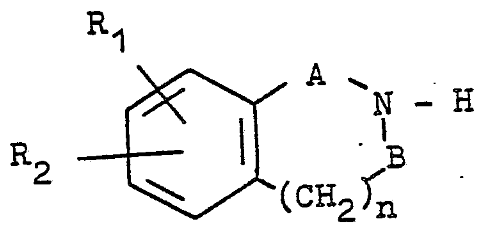

,(IV)

in der
$R_1$, $R_2$ und n wie eingangs definiert sind,
A eine Carbonylgruppe und
B eine Methylengruppe darstellt, durch Cyclisierung einer entsprechenden Verbindung der allgemeinen Formel

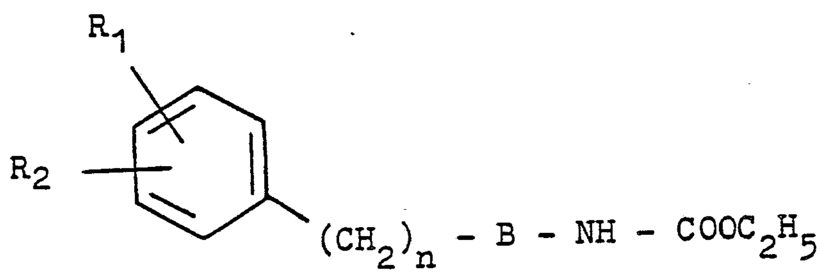

,(XII)

mit Polyphosphorsäure oder eine Verbindung der allgemeinen Formel IV, in der $R_1$, $R_2$ und n wie eingangs definiert sind, A und B jeweils eine CO-Gruppe darstellt durch Cyclisierung eines entsprechenden Diamids. Eine so erhaltene Verbindung der allgemeinen Formel IV kann anschließend durch Reduktion in eine entsprechende Methylenverbindung übergeführt werden.

Die als Ausgangsstoffe verwendeten Verbindungen der allgemeinen Formeln II, VI und VIII-XI erhält man durch Umsetzung einer Verbindung der allgemeinen Formel IV mit einer entsprechenden Halogenverbindung oder einer entsprechenden Dihalogenverbindung und gegebenenfalls anschließende Umsetzung mit einem entsprechenden Amin.

Wie bereits eingangs erwähnt, weisen die neuen Verbindungen der allgemeinen Formel I und deren physiologisch verträgliche Säureadditionssalze mit anorganischen oder organischen

Säuren wertvolle pharmakologische Eigenschaften auf, insbesondere neben einer milden blutdrucksenkenden Wirkung und einer positiv inotropen Wirkung eine herzfrequenzsenkende Wirkung sowie eine Herabsetzung des $O_2$-Bedarfs des Herzens.

Beispielsweise wurden die Verbindungen

A = 2-[N-Methyl-N-(2-(benzo[b]thienyl-3)-ethyl)-3-amino-propyl]-6,7-dimethoxy-1-oxo-1,2,3,4-tetrahydro-isochinolin-hydrochlorid,

B = 2-[N-Methyl-N-(2-(thienyl-3)-ethyl)-3-amino-propyl]-5,6-dimethoxy-1,3-dihydro-1-oxo-isoindol-dihydrochlorid und

C = 2-[N-Methyl-N-(2-(benzo[b]thienyl-3)-ethyl)-3-amino-propyl]-5,6-dimethoxy-1,3-dihydro-1-oxo-isoindol-dihydrochlorid

auf ihre biologischen Eigenschaften wie folgt untersucht:

Bestimmung der positiv inotropen und herzfrequenzsenkenden Wirkung an isolierten Meerschweinchen-Vorhöfen:

Die Bestimmung der positiv inotropen Wirkung erfolgte als Wirkung auf die Kontraktilität isolierter Meerschweinchen-Vorhöfe. Hierzu wurden frisch entnommene Meerschweinchen-Herzvorhöfe in ein Organbad von 65 ml überführt, das mit Krebs-Henseleit-Lösung (1,8 mM $Ca^{++}$) von 37°C gefüllt war. Das Bad wurde mit Carbogen (95 % Sauerstoff und 5 % Kohlendioxid) durchperlt. Die Vorhöfe wurden mit 1 Hz elektrisch gereizt; ihre Vordehnung betrug 10 mN. Die Kontraktionen wurden isometrisch auf einem Grass-Polygraphen registriert. Nach einer Äquilibrierungszeit von 60 Minuten wurden die zu prüfenden Substanzen so zugegeben, daß eine Endkonzentration von $10^{-5}$M erreicht wurde.

Die Bestimmung der Wirkung auf die Herzfrequenz erfolgte an spontan schlagenden Meerschweinchen-Vorhöfen in ansonsten gleicher Versuchsanordnung wie oben beschrieben.

Die nachfolgende Tabelle enthält die Mittelwerte von jeweils zwei Messungen:

| Substanz | Zunahme der Kontraktionskraft in % | Änderung der Herzfrequenz in % |
|---|---|---|
| A | + 54 | - 21 |
| B | + 127 | - 17 |
| C | + 61 | - 41 |

Aufgrund ihrer pharmakologischen Eigenschaften eignen sich die erfindungsgemäß hergestellen Verbindungen zur Behandlung der Herzinsuffizienz und zur Prophylaxe und Therapie ischämischer Herzerkrankungen.

Die zur Erzielung einer entsprechenden Wirkung erforderliche Dosierung beträgt zweckmäßigerweise ein- bis zweimal täglich 0,2 bis 5 mg/kg Körpergewicht, vorzugsweise 0,5 bis 2 mg/kg Körpergewicht. Hierzu lassen sich die erfindungsgemäß hergestellten Verbindungen der allgemeinen Formel I sowie ihre physiologisch verträglichen Säureadditionssalze mit anorganischen oder organischen Säuren, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Carboxymethylcellulose oder fetthaltigen

Substanzen wie Hartfett oder deren geeigneten Gemischen, in übliche galenische Zubereitungen wie Tabletten, Dragees, Kapseln, Pulver, Suspensionen, Tropfen, Ampullen, Säfte oder Zäpfchen einarbeiten.

Die nachfolgenden Beispiele sollen die Erfindung näher erläutern:

Beispiel 1

2-[N-Methyl-N-(2-(thienyl-2)-ethyl)-3-amino-propyl]-6,7-dimethoxy-1-oxo-1,2,3,4-tetrahydro-isochinolin-hydrochlorid

0,85 g (3,0 mMol) 2-(N-Methyl-3-amino-propyl)-6,7-dimethoxy-1-oxo-1,2,3,4-tetrahydro-isochinolin, 0,60 g (3,1 mMol) 2-(2-Brom-ethyl)-thiophen und 5 ml Triethylamin werden in 10 ml trockenem Dimethylformamid gelöst und 4 Stunden auf 100°C erhitzt. Die Reaktionsmischung wird im Vakuum eingeengt und der Rückstand in einem Gemisch aus 2 molarer Natronlauge und Methylenchlorid gelöst. Die organische Phase wird abgetrennt, mit gesättigter Kochsalzlösung gewaschen, über Natriumsulfat getrocknet und im Vakuum eingeengt. Das Rohprodukt wird durch Säulenchromatographie (Adsorptionsmittel: Kieselgel, Elutionsmittel: Methylenchlorid/Methanol) = 10/1) gereinigt. Aus einer Lösung in Methanol wird mit etherischer Salzsäure das Hydrochlorid gefällt, welches aus Aceton kristallisiert.

Ausbeute: 0,40 g (31 % der Theorie),

Schmelzpunkt: 150-151°C

| | | | | | | | | | | |
|---|---|---|---|---|---|---|---|---|---|---|
| Ber.: | C | 59,35 | H | 6,88 | N | 6,59 | Cl | 8,34 | S | 7,54 |
| Gef.: | | 59,40 | | 6,73 | | 6,29 | | 8,11 | | 7,59 |

$R_f$-Wert (Base): 0,42 (Kieselgel, Methylenchlorid in $NH_3$-Atmosphäre)

Beispiel 2

2-[N-Methyl-N-(2-(indolyl-3)-ethyl)-3-amino-propyl]-5,6-dimethoxy-phthalimid

1,15 g (3,5 mMol) 2-(3-Brom-propyl)-5,6-dimethoxy-phthalimid werden in 15 ml absolutem Dimethylformamid unter leichtem

Erwärmen gelöst. Nach der Zugabe von 0,61 g (3,5 mMol) 3-(2-Methylamino-ethyl)-indol und 15 ml Triethylamin wird 8 Stunden unter Rückfluß erhitzt. Das Reaktionsgemisch wird im Vakuum zur Trockene eingedampft und in 8%iger Natronlauge und Methylenchlorid verteilt. Die organische Phase wird dreimal mit je 10 ml Wasser gewaschen, abgetrennt, mit Natriumsulfat getrocknet und eingeengt. Nach der Reinigung über eine Kieselgelsäule mit Methylenchlorid/Methanol als Elutionsmittel erhält man 0,9 g eines gelben Öls.

Ausbeute: 0,90 g (61 % der Theorie),

| | | | | | | |
|---|---|---|---|---|---|---|
| Ber.: | C | 68,39 | H | 6,46 | N | 9,97 |
| Gef.: | | 68,28 | | 6,50 | | 9,91 |

$R_f$-Wert: 0,47 (Kieselgel, Methylenchlorid/Methanol = 9/1)

## Beispiel 3

2-[N-Methyl-N-(2-(thienyl-2)-ethyl)-3-amino-propyl]-5,6-dimethoxy-1,3-dihydro-1-oxo-isoindol-hydrochlorid

1,5 g (3,9 mMol) 2-[N-Methyl-N-(2-(thienyl-2)-ethyl)-3-amino-propyl]-5,6-dimethoxy-phthalimid und 1,5 g (23 mMol) Zinkpulver werden in 15 ml Eisessig 1,5 Stunden zum Rückfluß erhitzt. Man filtriert noch heiß vom Ungelösten ab und extrahiert den Rückstand zweimal mit je 8 ml heißem Eisessig. Die gesammelten Essigsäurephasen werden im Vakuum eingedampft und der Rückstand in 8%iger Natronlauge und Methylenchlorid verteilt. Die organische Phase wird mit Wasser gewaschen, abgetrennt, über Natriumsulfat getrocknet und im Vakuum eingeengt. Die Reinigung des Rohprodukts über eine Kieselgelsäule mit Methylenchlorid/Methanol als Eluens liefert 1,1 g (75 % der Theorie) eines gelben Öls. Es wird in wenig absolutem Methanol gelöst und mit etherischer Salzsäure das Hydrochlorid gefällt.

Ausbeute: 1,0 g (62 % der Theorie),

Schmelzpunkt: 211-212°C

| | | | | | | | | | | |
|---|---|---|---|---|---|---|---|---|---|---|
| Ber.: | C | 58,45 | H | 6,62 | N | 6,82 | Cl | 8,63 | S | 7,80 |
| Gef.: | | 58,68 | | 6,80 | | 7,00 | | 8,84 | | 7,59 |

$R_f$-Wert (Base): 0,55 (Kieselgel, Methylenchlorid/Methanol = 9/1)

Beispiel 4

2-[N-Methyl-N-(3-(pyridyl-4)-propyl)-3-amino-propyl]-6,7-methylendioxy-1-oxo-1,2,3,4-tetrahydro-isochinolin-dihydrochlorid

1,3 g (5 mMol) 2-(2-Formyl-ethyl)-6,7-methylendioxy-1-oxo-1,2,3,4-tetrahydro-isochinolin und 0,75 g (5 mMol) 4-(3-Methylamino-propyl)-pyridin werden in 50 ml Ethanol in Gegenwart von 0,2 g 10%igem Palladium auf Aktivkohle bei 70°C und 5 bar bis zur Aufnahme der berechneten Menge Wasserstoff hydriert. Der Katalysator wird abfiltriert und das Filtrat im Vakuum zur Trockne eingedampft. Aus einer Lösung in Aceton wird mit etherischer Salzsäure das Dihydrochlorid gefällt, welches aus Aceton/Ether umkristallisiert wird.
Ausbeute: 1,7 g (74 % der Theorie),
Schmelzpunkt: 86-94°C

| | | | | | | | | |
|---|---|---|---|---|---|---|---|---|
| Ber.: | C | 58,15 | H | 6,43 | N | 9,24 | Cl | 15,60 |
| Gef.: | | 57,91 | | 6,31 | | 9,15 | | 15,36 |

Die Reduktion kann auch mit Natriumborhydrid in Ethanol bei Raumtemperatur oder Siedetemperatur durchgeführt werden.

Beispiel 5

2-[N-Methyl-N-(3-(pyridyl-4)-propyl)-3-amino-propyl]-5,6-dimethyl-1,3-dihydro-isoindol-dihydrochlorid

Ein Gemisch aus 2,0 g (5,7 mMol) 2-[N-Methyl-N-(3-pyridyl-4)-

propyl)-3-amino-propyl]-5,6-dimethyl-1,3-dihydro-1-oxo-isoindol und 0,43 g (11,4 mMol) Lithiumaluminiumhydrid in 45 ml absolutem Tetrahydrofuran wird eine Stunde unter Rückfluß erhitzt. Nach dem Abkühlen wird mit 0,57 ml Wasser, 0,57 ml 10%iger Natronlauge und mit 1,71 ml Wasser zersetzt, abgesaugt und das Filtrat im Vakuum eingedampft. Der erhaltene Rückstand wird über 180 g Aluminiumoxid (neutral, Aktivität II-III) mit Methylenchlorid und anschließend mit steigenden Anteilen von Ethanol (bis 3 %) gereinigt. Aus einer Lösung in Aceton wird mit etherischer Salzsäure das Dihydrochlorid gefällt.

Ausbeute: 1,85 g (80 % der Theorie),

Schmelzpunkt: 239-241°C

| | | | | | | | | |
|---|---|---|---|---|---|---|---|---|
| Ber.: | C | 64,37 | H | 8,10 | N | 10,23 | Cl | 17,27 |
| Gef.: | | 64,20 | | 8,37 | | 10,07 | | 17,36 |

## Beispiel 6

**2-[N-Methyl-N-(2-(6,7-dimethoxy-isochinolyl-4)-ethyl)-3-amino-propyl]-6,7-dimethoxy-1-oxo-1,2,3,4-tetrahydro-isochinolin-dihydrochlorid**

a) 2-[N-Methyl-N-(2-(6,7-dimethoxy-isochinolyl-4)-acetyl)-3-amino-propyl]-6,7-dimethoxy-1-oxo-1,2,3,4-tetrahydro-isochinolin-semihydrat

Zu einer Lösung von 4,9 g (17 mMol) 6,7-Dimethoxy-isochinolyl-4-essigsäure in 100 ml Dimethylformamid werden 3,2 g (20 mMol) N,N'-Carbonyl-diimidazol gegeben. Nach ca. 30 Minuten werden 4,8 g (17 mMol) 2-(3-Methylamino-propyl)-6,7-dimethoxy-1-oxo-1,2,3,4-tetrahydro-isochinolin zugefügt und 2 Stunden bei Raumtemperatur gerührt. Das Lösungsmittel wird im Vakuum abgedampft und der verbliebene Rückstand in einem Gemisch aus 2 molarer Natronlauge und Methylenchlorid

gelöst. Die organische Phase wird mit Wasser gewaschen, über Magnesiumsulfat getrocknet und im Vakuum eingedampft.
Ausbeute: 5,3 g (63 % der Theorie),
Schmelzpunkt: 80-85°C

| | | | | | | |
|---|---|---|---|---|---|---|
| Ber.: | C | 65,10 | H | 6,63 | N | 8,13 |
| Gef.: | | 64,90 | | 6,63 | | 8,15 |

b) <u>2-[N-Methyl-N-(2-(6,7-dimethoxy-isochinolyl-4)-ethyl)-3-amino-propyl]-6,7-dimethoxy-1-oxo-1,2,3,4-tetrahydro-isochinolin-dihydrochlorid</u>

Zu einer Lösung von 4,1 g (8 mMol) 2-[N-Methyl-N-(2-(6,7-dimethoxy-isochinolyl-4)-acetyl)-3-amino-propyl]-6,7-dimethoxy-1-oxo-1,2,3,4-tetrahydro-isochinolin-semihydrat in 250 ml Tetrahydrofuran werden 12 ml 1 molarer Boran-Tetrahydrofuran-Komplex in Tetrahydrofuran zugegeben und unter Rühren bei Raumtemperatur 1,5 ml (12 mMol) Bortrifluorid-diethyletherat-Komplex zugetropft. Nach 3 Stunden Reaktionszeit wird tropfenweise mit 15 ml 6 molarer Salzsäure versetzt, 0,5 Stunden am Rückfluß erhitzt und danach das Lösungsmittel im Vakuum abgedampft. Der verbliebene wässrige Teil wird mit 2 molarer Natronlauge alkalisch gemacht und mit Methylenchlorid ausgeschüttelt. Die organische Phase wird mit Wasser gewaschen, über Natriumsulfat getrocknet, im Vakuum eingedampft und über eine Kieselgelsäule (0,063-0,2 mm) mit Methylenchlorid und anschließend steigenden Anteilen von Ethanol (bis 5 %) gereinigt. Aus einer Lösung in Aceton wird mit etherischer Salzsäure das Dihydrochlorid gefällt, welches aus Aceton umkristallisiert wird.
Ausbeute: 0,45 g (10 % der Theorie),
Schmelzpunkt: 158-169°C

| | | | | | | | | |
|---|---|---|---|---|---|---|---|---|
| Ber.: | C | 59,36 | H | 6,58 | N | 7,42 | Cl | 12,52 |
| Gef.: | | 59,20 | | 6,60 | | 7,27 | | 12,22 |

Die Reduktion kann auch mit Lithiumaluminiumhydrid in Ether oder Tetrahydrofuran bei Rückflußtemperatur durchgeführt werden.

Beispiel 7

2-[N-Methyl-N-(4-(thienyl-2)-butyl)-3-amino-propyl]-6,7-dimethoxy-1-oxo-1,2,3,4-tetrahydro-isochinolin-hydrochlorid

Hergestellt aus 2-(N-Methyl-3-amino-propyl)-6,7-dimethoxy-1-oxo-1,2,3,4-tetrahydro-isochinolin und 2-(4-Brom-butyl)-thiophen analog Beispiel 1.
Ausbeute: 64 % der Theorie,
Schmelzpunkt: 115-120°C

| | | | | | | | | | | |
|---|---|---|---|---|---|---|---|---|---|---|
| Ber.: | C | 60,98 | H | 7,34 | N | 6,18 | Cl | 7,83 | S | 7,08 |
| Gef.: | | 60,70 | | 7,44 | | 5,96 | | 8,15 | | 7,03 |

$R_f$-Wert: 0,29 (Kieselgel, Methylenchlorid in $NH_3$-Atmosphäre)

Beispiel 8

2-[N-Methyl-N-(2-(benzo[b]furyl-2)-ethyl)-3-amino-propyl]-6,7-dimethoxy-1-oxo-1,2,3,4-tetrahydro-isochinolin-hydrochlorid

Hergestellt aus 2-(N-Methyl-3-amino-propyl)-6,7-dimethoxy-1-oxo-1,2,3,4-tetrahydro-isochinolin und 2-(2-Chlor-ethyl)-benzo[b]furan analog Beispiel 1.
Ausbeute: 24 % der Theorie,
Schmelzpunkt: 180-184°C

| | | | | | | | | |
|---|---|---|---|---|---|---|---|---|
| Ber.: | C | 65,42 | H | 6,81 | N | 6,10 | Cl | 7,72 |
| Gef.: | | 65,40 | | 6,80 | | 5,92 | | 7,98 |

Beispiel 9

2-[N-Methyl-N-(2-(benzo[b]thienyl-3)-ethyl)-3-amino-propyl]-6,7-dimethoxy-1-oxo-1,2,3,4-tetrahydro-isochinolin-hydrochlorid

Hergestellt aus 2-(N-Methyl-3-amino-propyl)-6,7-dimethoxy-1-oxo-1,2,3,4-tetrahydro-isochinolin und 3-(2-Methylsulfonyloxy-ethyl)-benzo[b]thiophen analog Beispiel 1.
Ausbeute: 38 % der Theorie,

| | | | | | | | | | | |
|---|---|---|---|---|---|---|---|---|---|---|
| Ber.: | C | 63,21 | H | 6,58 | N | 5,90 | Cl | 7,46 | S | 6,75 |
| Gef.: | | 63,10 | | 6,47 | | 5,86 | | 7,55 | | 6,88 |

$R_f$-Wert: 0,37 (Kieselgel, Methylenchlorid in $NH_3$-Atmosphäre)

Beispiel 10

2-[N-Methyl-N-(2-(thienyl-3)-ethyl)-3-amino-propyl]-6,7-dimethoxy-1-oxo-1,2,3,4-tetrahydro-isochinolin-hydrochlorid

Hergestellt aus 2-(N-Methyl-3-amino-propyl)-6,7-dimethoxy-1-oxo-1,2,3,4-tetrahydro-isochinolin und 3-(2-Brom-ethyl)-thiophen analog Beispiel 1.
Ausbeute: 34 % der Theorie,

| | | | | | | | | | | |
|---|---|---|---|---|---|---|---|---|---|---|
| Ber.: | C | 59,35 | H | 6,88 | N | 6,59 | Cl | 8,34 | S | 7,54 |
| Gef.: | | 59,47 | | 6,77 | | 6,57 | | 8,59 | | 7,37 |

$R_f$-Wert: 0,34 (Kieselgel, Methylenchlorid in $NH_3$-Atmosphäre)

Beispiel 11

2-[N-Methyl-N-(2-(indolyl-3)-ethyl)-3-amino-propyl]-6,7-dimethoxy-1-oxo-1,2,3,4-tetrahydro-isochinolin-hydrochlorid

Hergestellt aus 2-(N-Methyl-3-amino-propyl)-6,7-dimethoxy-1-oxo-1,2,3,4-tetrahydro-isochinolin und 3-(2-Chlor-ethyl)-indol analog Beispiel 1.
Ausbeute: 32 % der Theorie,

| | | | | | | | | |
|---|---|---|---|---|---|---|---|---|
| Ber.: | C | 65,56 | H | 7,04 | N | 9,17 | Cl | 7,74 |
| Gef.: | | 65,38 | | 6,93 | | 9,01 | | 7,97 |

$R_f$-Wert: 0,45 (Kieselgel, Methylenchlorid/Methanol = 10/1 in $NH_3$-Atmosphäre)

Beispiel 12

2-[N-Methyl-N-(3-(indolyl-3)-propyl)-3-amino-propyl]-6,7-dimethoxy-1-oxo-1,2,3,4-tetrahydro-isochinolin-hydrochlorid

Hergestellt aus 2-(N-Methyl-3-amino-propyl)-6,7-dimethoxy-1-oxo-1,2,3,4-tetrahydro-isochinolin und 3-(3-Methylsulfonyloxy-propyl)-indol analog Beispiel 1.
Ausbeute: 38 % der Theorie,

| | | | | | | | | |
|---|---|---|---|---|---|---|---|---|
| Ber.: | C | 66,16 | H | 7,26 | N | 8,90 | Cl | 7,51 |
| Gef.: | | 65,98 | | 7,56 | | 8,85 | | 7,54 |

$R_f$-Wert: 0,34 (Kieselgel, Methylenchlorid/Methanol = 10/1 in $NH_3$-Atmosphäre)

Beispiel 13

2-[N-Methyl-N-(4-(thienyl-2)-butyl)-3-amino-propyl]-5,6-dimethoxy-phthalimid

Hergestellt aus 2-(3-Methylamino-propyl)-5,6-dimethoxy-phthalimid und 2-(4-Brom-butyl)-thiophen analog Beispiel 1.
Ausbeute: 80 % der Theorie,

| | | | | | | | | |
|---|---|---|---|---|---|---|---|---|
| Ber.: | C | 63,44 | H | 6,78 | N | 6,73 | S | 7,70 |
| Gef.: | | 63,50 | | 6,71 | | 6,75 | | 7,90 |

$R_f$-Wert: 0,57 (Kieselgel, Methylenchlorid/Methanol = 9/1)

Beispiel 14

2-[N-Methyl-N-(3-(indolyl-3)-propyl)-3-amino-propyl]-5,6-dimethoxy-phthalimid

Hergestellt aus 2-(3-Methylamino-propyl)-5,6-dimethoxy-phthalimid und 3-(3-Methylsulfonyloxy-propyl)-indol analog Beispiel 1.
Ausbeute: 51 % der Theorie,

| | | | | | | |
|---|---|---|---|---|---|---|
| Ber.: | C | 68,95 | H | 6,71 | N | 9,65 |
| Gef.: | | 69,20 | | 6,78 | | 9,60 |

$R_f$-Wert: 0,36 (Kieselgel, Methylenchlorid/Methanol = 9/1)

Beispiel 15

2-[N-Methyl-N-(2-(thienyl-3)-ethyl)-3-amino-propyl]-5,6-dimethoxy-phthalimid

Hergestellt aus 2-(3-Methylamino-propyl)-5,6-dimethoxy-phthalimid und 3-(2-Brom-ethyl)-thiophen analog Beispiel 1.

Ausbeute: 73 % der Theorie,

| | | | | | | | | |
|---|---|---|---|---|---|---|---|---|
| Ber.: | C | 61,84 | H | 6,23 | N | 7,21 | S | 8,25 |
| Gef.: | | 61,64 | | 6,28 | | 7,19 | | 8,09 |

$R_f$-Wert: 0,48 (Kieselgel, Methylenchlorid/Methanol = 9/1)

## Beispiel 16

2-[N-Methyl-N-(2-(benzo[b]thienyl-3)-ethyl)-3-amino-propyl]-5,6-dimethoxy-phthalimid

Hergestellt aus 2-(3-Methylamino-propyl)-5,6-dimethoxy-phthalimid und 3-(2-Methylsulfonyloxy-ethyl)-benzo[b]thiophen analog Beispiel 1.

Ausbeute: 41 % der Theorie,

| | | | | | | | | |
|---|---|---|---|---|---|---|---|---|
| Ber.: | C | 65,73 | H | 5,98 | N | 6,39 | S | 7,31 |
| Gef.: | | 65,78 | | 5,80 | | 6,19 | | 7,42 |

$R_f$-Wert: 0,61 (Kieselgel, Methylenchlorid/Methanol = 9/1)

## Beispiel 17

2-[N-Methyl-N-(2-(thienyl-2)-ethyl)-3-amino-propyl]-5,6-dimethoxy-phthalimid

Hergestellt aus 2-(3-Methylamino-propyl)-5,6-dimethoxy-phthalimid und 3-(2-Brom-ethyl)-thiophen analog Beispiel 1.

Ausbeute: 44 % der Theorie,

| | | | | | | | | |
|---|---|---|---|---|---|---|---|---|
| Ber.: | C | 61,84 | H | 6,23 | N | 7,21 | S | 8,25 |
| Gef.: | | 61,59 | | 6,20 | | 7,02 | | 8,42 |

$R_f$-Wert: 0,24 (Kieselgel, Methylenchlorid/Methanol = 9/1)

Beispiel 18

2-[N-Methyl-N-(3-(pyridyl-3)-propyl)-3-amino-propyl]-6,7-dimethoxy-1-oxo-1,2,3,4-tetrahydro-isochinolin-dihydrochlorid-monohydrat

Hergestellt aus 2-(3-Methylamino-propyl)-6,7-dimethoxy-1-oxo-1,2,3,4-tetrahydro-isochinolin und 3-(3-Chlor-propyl)-pyridin analog Beispiel 1.

Ausbeute: 31 % der Theorie,

| | | | | | | | | |
|---|---|---|---|---|---|---|---|---|
| Ber.: | C | 56,55 | H | 7,22 | N | 8,60 | Cl | 14,51 |
| Gef.: | | 56,53 | | 7,49 | | 8,45 | | 14,71 |

$R_f$-Wert: 0,20 (Kieselgel, Essigester/Ethanol/Ammoniak = 80/40/2)

Beispiel 19

2-[N-Methyl-N-(3-(pyridyl-3-N-oxid)-propyl)-3-amino-propyl]-6,7-dimethoxy-1-oxo-1,2,3,4-tetrahydro-isochinolin-dihydrochlorid

Hergestellt aus 2-(3-Methylamino-propyl)-6,7-dimethoxy-1-oxo-1,2,3,4-tetrahydro-isochinolin und 3-(3-Chlor-propyl)-pyridin-N-oxid analog Beispiel 1.

Ausbeute: 33 % der Theorie,

| | | | | | | | | |
|---|---|---|---|---|---|---|---|---|
| Ber.: | C | 57,02 | H | 6,45 | N | 8,67 | Cl | 14,63 |
| Gef.: | | 57,07 | | 6,23 | | 8,44 | | 14,57 |

$R_f$-Wert: 0,35 (Kieselgel, Essigester/Ethanol/Ammoniak = 50/45/5)

Beispiel 20

2-[N-Methyl-N-(2-(indolyl-3)-ethyl)-3-amino-propyl]-5,6-dimethoxy-1,3-dihydro-1-oxo-isoindol-hydrochlorid

Hergestellt aus 2-[N-Methyl-N-(2-(indolyl-3)-ethyl)-3-amino-propyl)-5,6-dimethoxy-phthalimid analog Beispiel 3.
Ausbeute: 25 % der Theorie,

| | | | | | | | | |
|---|---|---|---|---|---|---|---|---|
| Ber.: | C | 64,93 | H | 6,81 | N | 9,47 | Cl | 7,99 |
| Gef.: | | 64,80 | | 6,81 | | 9,41 | | 8,03 |

$R_f$-Wert: 0,29 (Kieselgel, Methylenchlorid/Methanol = 9/1)

Beispiel 21

2-[N-Methyl-N-(3-(indolyl-3)-propyl)-3-amino-propyl]-5,6-dimethoxy-1,3-dihydro-1-oxo-isoindol-hydrochlorid

Hergestellt aus 2-[N-Methyl-N-(3-(indolyl-3)-propyl)-3-amino-propyl)-5,6-dimethoxy-phthalimid analog Beispiel 3.
Ausbeute: 24 % der Theorie,

| | | | | | | | | |
|---|---|---|---|---|---|---|---|---|
| Ber.: | C | 65,56 | H | 7,04 | N | 9,18 | Cl | 7,74 |
| Gef.: | | 65,41 | | 7,24 | | 8,86 | | 7,91 |

$R_f$-Wert: 0,36 (Kieselgel, Methylenchlorid/Methanol = 6/4)

Beispiel 22

2-[N-Methyl-N-(2-(thienyl-3)-ethyl)-3-amino-propyl]-5,6-dimethoxy-1,3-dihydro-1-oxo-isoindol-dihydrochlorid

Hergestellt aus 2-[N-Methyl-N-(2-(thienyl-3)-ethyl)-3-amino-propyl)-5,6-dimethoxy-phthalimid analog Beispiel 3.

Ausbeute: 70 % der Theorie,
Schmelzpunkt: 187-188°C

| | | | | | | | | | | |
|---|---|---|---|---|---|---|---|---|---|---|
| Ber.: | C | 53,69 | H | 6,31 | N | 6,30 | Cl | 15,85 | S | 7,16 |
| Gef.: | | 53,54 | | 6,49 | | 6,48 | | 15,78 | | 7,17 |

$R_f$-Wert: 0,43 (Kieselgel, Methylenchlorid/Methanol = 9/1)

## Beispiel 23

**2-[N-Methyl-N-(2-(benzo[b]thienyl-3)-ethyl)-3-amino-propyl]-5,6-dimethoxy-1,3-dihydro-1-oxo-isoindol-dihydrochlorid**

Hergestellt aus 2-[N-Methyl-N-(2-(benzo[b]thienyl-3)-ethyl)-3-amino-propyl)-5,6-dimethoxy-phthalimid analog Beispiel 3.
Ausbeute: 63 % der Theorie,

| | | | | | | | | | | |
|---|---|---|---|---|---|---|---|---|---|---|
| Ber.: | C | 57,94 | H | 6,08 | N | 5,63 | Cl | 14,25 | S | 6,44 |
| Gef.: | | 58,19 | | 6,25 | | 5,82 | | 14,32 | | 6,26 |

$R_f$-Wert: 0,43 (Kieselgel, Methylenchlorid/Methanol = 9/1)

## Beispiel 24

**2-[N-Methyl-N-(3-(pyridyl-4)-propyl)-3-amino-propyl]-5,6-dimethyl-1,3-dihydro-1-oxo-isoindol**

Hergestellt aus 2-[N-Methyl-N-(3-(pyridyl-4)-propyl)-3-amino-propyl]-5,6-dimethyl-phthalimid analog Beispiel 3.
Ausbeute: 80 % der Theorie,
Schmelzpunkt: 82-84°C

| | | | | | | |
|---|---|---|---|---|---|---|
| Ber.: | C | 75,17 | H | 8,32 | N | 11,95 |
| Gef.: | | 74,98 | | 8,39 | | 11,76 |

Beispiel 25

2-[N-Methyl-N-(3-(pyridyl-3)-propyl)-3-amino-propyl]-5,6-dimethoxy-1,3-dihydro-1-oxo-isoindol-hydrochlorid

Hergestellt aus 2-[N-Methyl-N-(3-(pyridyl-3)-propyl)-3-amino-propyl]-5,6-dimethoxy-phthalimid analog Beispiel 3.

Ausbeute: 60 % der Theorie,

Schmelzpunkt: 180-182°C

| | | | | | | | | |
|---|---|---|---|---|---|---|---|---|
| Ber.: | C | 62,91 | H | 7,20 | N | 10,00 | Cl | 8,44 |
| Gef.: | | 62,75 | | 7,09 | | 9,85 | | 8,14 |

Beispiel 26

2-[N-Methyl-N-((pyridyl-3)-methyl)-3-amino-propyl]-5,6-dimethoxy-1,3-dihydro-1-oxo-isoindol-dihydrochlorid

Hergestellt aus 2-[N-Methyl-N-((pyridyl-3)-methyl)-3-amino-propyl]-5,6-dimethoxy-phthalimid analog Beispiel 3.

Ausbeute: 72 % der Theorie,

| | | | | | | | | |
|---|---|---|---|---|---|---|---|---|
| Ber.: | C | 56,07 | H | 6,35 | N | 9,81 | Cl | 16,55 |
| Gef.: | | 56,16 | | 6,38 | | 9,85 | | 16,44 |

$R_f$-Wert: 0,35 (Kieselgel, Essigester/Ethanol/Ammoniak = 90/10/1)

Beispiel 27

2-[N-Methyl-N-(2-(6,7-dimethoxy-isochinolyl-4)-ethyl)-3-amino-propyl]-5,6-dimethoxy-1,3-dihydro-1-oxo-isoindol-hydrochlorid

Hergestellt aus 2-[N-Methyl-N-(2-(6,7-dimethoxy-isochinolyl-4)-ethyl)-3-amino-propyl]-5,6-dimethoxy-phthalimid analog Beispiel 3.
Ausbeute: 90 % der Theorie,
Schmelzpunkt: 202-204°C

| | | | | | | | | |
|---|---|---|---|---|---|---|---|---|
| Ber.: | C | 62,84 | H | 6,64 | N | 8,14 | Cl | 6,87 |
| Gef.: | | 62,61 | | 6,78 | | 7,95 | | 6,59 |

$R_f$-Wert: 0,50 (Kieselgel, Essigester/Ethanol/Ammoniak = 50/45/5)

Beispiel 28

2-[N-Methyl-N-(2-(6,7-dimethoxy-isochinolyl-4)-ethyl)-3-amino-propyl]-5,6-dimethyl-1,3-dihydro-1-oxo-isoindol-dihydrochlorid-monohydrat

Hergestellt aus 2-[N-Methyl-N-(2-(6,7-dimethoxy-isochinolyl-4)-ethyl)-3-amino-propyl]-5,6-dimethyl-phthalimid analog Beispiel 3.
Ausbeute: 81 % der Theorie,
Schmelzpunkt: 227-229°C

| | | | | | | | | |
|---|---|---|---|---|---|---|---|---|
| Ber.: | C | 60,22 | H | 6,92 | N | 7,80 | Cl | 13,16 |
| Gef.: | | 60,40 | | 7,03 | | 8,05 | | 13,08 |

Beispiel 29

2-[N-Methyl-N-((pyridyl-3)-methyl)-3-amino-propyl]-5,6-dimethyl-1,3-dihydro-1-oxo-isoindol

Hergestellt aus 2-[N-Methyl-N-((pyridyl-3)-methyl)-3-amino-propyl]-5,6-dimethyl-phthalimid analog Beispiel 3.
Ausbeute: 73 % der Theorie,
Schmelzpunkt: 93-95°C

| | | | | | | |
|---|---|---|---|---|---|---|
| Ber.: | C | 74,27 | H | 7,79 | N | 12,99 |
| Gef.: | | 74,27 | | 7,73 | | 12,92 |

Beispiel 30

2-[N-Methyl-N-((pyridyl-3)-methyl)-3-amino-propyl]-6,7-methylendioxy-1-oxo-1,2,3,4-tetrahydro-isochinolin-hydrochlorid

Hergestellt aus 2-(2-Formyl-ethyl)-6,7-methylendioxy-1-oxo-1,2,3,4-tetrahydro-isochinolin und 3-(Methylamino-methyl)-pyridin analog Beispiel 4.
Ausbeute: 83 % der Theorie,
Schmelzpunkt: 203-205°C

| | | | | | | | | |
|---|---|---|---|---|---|---|---|---|
| Ber.: | C | 61,61 | H | 6,20 | N | 10,79 | Cl | 9,09 |
| Gef.: | | 61,72 | | 6,18 | | 10,62 | | 8,96 |

Beispiel 31

2-[N-Methyl-N-((pyridyl-3)-methyl)-3-amino-propyl]-5,6-dimethyl-phthalimid

Hergestellt aus 2-(2-Formyl-ethyl)-5,6-dimethyl-phthalimid und 3-(Methylamino-methyl)-pyridin analog Beispiel 4.
Ausbeute: 80 % der Theorie,
Schmelzpunkt: 71-72°C

| | | | | | | |
|---|---|---|---|---|---|---|
| Ber.: | C | 71,19 | H | 6,87 | N | 12,45 |
| Gef.: | | 70,98 | | 6,77 | | 12,32 |

Beispiel 32

2-[N-Methyl-N-(3-(pyridyl-4)-propyl)-3-amino-propyl]-5,6-dimethyl-phthalimid-dihydrochlorid-semihydrat

Hergestellt aus 2-(2-Formyl-ethyl)-5,6-dimethyl-phthalimid und 4-(3-Methylamino-propyl)-pyridin analog Beispiel 4.
Ausbeute: 68 % der Theorie,

| | | | | | | | | |
|---|---|---|---|---|---|---|---|---|
| Ber.: | C | 59,05 | H | 6,76 | N | 9,39 | Cl | 15,84 |
| Gef.: | | 58,95 | | 6,86 | | 9,11 | | 15,64 |

$R_f$-Wert: 0,60 (Kieselgel, Essigester/Ethanol/Ammoniak = 90/10/1)

Beispiel 33

2-[N-Methyl-N-(2-(6,7-dimethoxy-isochinolyl-4)-ethyl)-3-amino-propyl]-5,6-dimethyl-phthalimid

Hergestellt aus 2-(2-Formyl-ethyl)-5,6-dimethyl-phthalimid und 4-(2-Methylamino-ethyl)-6,7-dimethoxy-isochinolin analog Beispiel 4.
Ausbeute: 76 % der Theorie,
Schmelzpunkt: 122-124°C

| | | | | | | |
|---|---|---|---|---|---|---|
| Ber.: | C | 70,26 | H | 6,77 | N | 9,10 |
| Gef.: | | 70,43 | | 6,85 | | 8,98 |

Beispiel 34

2-[N-Methyl-N-(2-(pyridyl-2)-ethyl)-3-amino-propyl]-5,6-dimethoxy-phthalimid

Hergestellt aus 2-(2-Formyl-ethyl)-5,6-dimethoxy-phthalimid

und 2-(2-Methylamino-ethyl)-pyridin analog Beispiel 4.
Ausbeute: 63 % der Theorie,
Schmelzpunkt: 120-122°C

| | | | | | | |
|---|---|---|---|---|---|---|
| Ber.: | C | 65,78 | H | 6,58 | N | 10,96 |
| Gef.: | | 65,90 | | 6,55 | | 11,11 |

Beispiel 35

2-[N-Methyl-N-(2-(6,7-dimethoxy-isochinolyl-4)-ethyl)-3-amino-propyl]-5,6-dimethoxy-phthalimid-semihydrat

Hergestellt aus 2-(2-Formyl-ethyl)-5,6-dimethoxy-phthalimid und 4-(2-Methylamino-ethyl)-6,7-dimethoxy-isochinolin analog Beispiel 4.
Ausbeute: 75 % der Theorie,
Schmelzpunkt: 158-160°C

| | | | | | | |
|---|---|---|---|---|---|---|
| Ber.: | C | 64,52 | H | 6,42 | N | 8,36 |
| Gef.: | | 64,44 | | 6,46 | | 8,44 |

Beispiel 36

2-[N-Methyl-N-(2-(6,7-dimethoxy-isochinolyl-4)-ethyl)-3-amino-propyl]-6,7-methylendioxy-1-oxo-1,2,3,4-tetrahydro-isochinolin-dihydrochlorid-semihydrat

Hergestellt aus 2-(2-Formyl-ethyl)-6,7-methylendioxy-1-oxo-1,2,3,4-tetrahydro-isochinolin und 4-(2-Methylamino-ethyl)-6,7-dimethoxy-isochinolin analog Beispiel 4.
Ausbeute: 77 % der Theorie,

| | | | | | | | | |
|---|---|---|---|---|---|---|---|---|
| Ber.: | C | 57,96 | H | 6,12 | N | 7,51 | Cl | 12,67 |
| Gef.: | | 57,85 | | 6,06 | | 7,34 | | 12,82 |

$R_f$-Wert: 0,25 (Kieselgel, Essigester/Ethanol/Ammoniak = 80/40/2)

Beispiel 37

2-[N-Methyl-N-(2-(6,7-dimethoxy-isochinolyl-4)-ethyl)-3-amino-propyl]-6,7-methylendioxy-1,2,3,4-tetrahydro-isochinolin-trihydrochlorid-dihydrat

Hergestellt aus 2-[N-Methyl-N-(2-(6,7-dimethoxy-isochinolyl-4)-ethyl)-3-amino-propyl]-6,7-methylendioxy-1-oxo-1,2,3,4-tetrahydro-isochinolin analog Beispiel 5.

Ausbeute: 83 % der Theorie,

| | | | | | | | | |
|---|---|---|---|---|---|---|---|---|
| Ber.: | C | 53,24 | H | 6,62 | N | 6,90 | Cl | 17,46 |
| Gef.: | | 53,06 | | 6,66 | | 6,91 | | 17,53 |

$R_f$-Wert: 0,15 (Kieselgel, Methylenchlorid/Ethanol = 4/1)

Beispiel 38

2-[N-Methyl-N-(3-(pyridyl-4)-propyl)-3-amino-propyl]-5,6-dimethoxy-1,3-dihydro-isoindol-dihydrochlorid-monohydrat

Hergestellt aus 2-[N-Methyl-N-(3-(pyridyl-4)-propyl)-3-amino-propyl]-5,6-dimethoxy-1,3-dihydro-1-oxo-isoindol analog Beispiel 5.

Ausbeute: 55 % der Theorie,

Schmelzpunkt: 248-250°C

| | | | | | | | | |
|---|---|---|---|---|---|---|---|---|
| Ber.: | C | 57,38 | H | 7,66 | N | 9,12 | Cl | 15,40 |
| Gef.: | | 57,28 | | 7,52 | | 9,02 | | 15,50 |

Beispiel 39

2-[N-Methyl-N-(4-(thienyl-2)-butyl)-3-amino-propyl]-5,6-dimethoxy-1,3-dihydro-1-oxo-isoindol-hydrochlorid

Hergestellt aus 2-[N-Methyl-N-(4-(thienyl-2)-butyl)-3-amino-propyl]-5,6-dimethoxy-phthalimid analog Beispiel 3.
Ausbeute: 30 % der Theorie,
Schmelzpunkt: 171-172°C (Zers.)

| | | | | | | | | | | |
|---|---|---|---|---|---|---|---|---|---|---|
| Ber.: | C | 60,19 | H | 7,12 | N | 6,38 | Cl | 8,08 | S | 7,30 |
| Gef.: | | 59,92 | | 7,21 | | 6,44 | | 8,29 | | 7,41 |

Beispiel 40

2-[N-Methyl-N-(4-(thienyl-2)-butyl)-3-amino-propyl]-6,7-dimethoxy-1-oxo-1,2,3,4-tetrahydro-isochinolin-hydrochlorid

0,83 g (4,0 mMol) 6,7-Dimethoxy-1-oxo-1,2,3,4-tetrahydro-isochinolin werden in 15 ml Dimethylformamid gelöst und unter Rühren mit 0,49 g (4,4 mMol) Kalium-tert.butylat versetzt. Nach Abklingen der exothermen Reaktion fällt das Kaliumsalz aus. Man kühlt auf 0°C ab, fügt 1,3 g (4,4 mMol) N-Methyl-N-(4-(thienyl-2)-butyl)-3-amino-1-brom-propan zu und läßt bei 0°C 4 Stunden reagieren. Nach Zersetzen mit Eiswasser wird mit Essigester extrahiert, die organische Phase mit Wasser gewaschen, über Magnesiumsulfat getrocknet, eingedampft und über eine Kieselgelsäule gereinigt. Die so erhaltene freie Base wird in Aceton gelöst und mit etherischer Salzsäure das Hydrochlorid gefällt.
Ausbeute: 0,63 g (35 % der Theorie),
Schmelzpunkt: 116-119°C (Zers.)

| | | | | | | | | | | |
|---|---|---|---|---|---|---|---|---|---|---|
| Ber.: | C | 60,98 | H | 7,34 | N | 6,18 | Cl | 7,83 | S | 7,08 |
| Gef.: | | 61,15 | | 7,38 | | 6,08 | | 7,68 | | 6,97 |

Beispiel 41

2-[N-Methyl-N-(2-(benzo[b]thienyl-3)-ethyl)-3-amino-propyl]-6,7-dimethoxy-1-thioxo-1,2,3,4-tetrahydro-isochinolin

1,7 g (3,9 mMol) 2-[N-Methyl-N-(2-(benzo[b]thienyl-3)-ethyl)-3-amino-propyl]-6,7-dimethoxy-1-oxo-1,2,3,4-tetrahydro-isochinolin und 0,57 g (2,0 mMol) 2,4-Bis-(methylthio)-1,3-dithia-2,4-diphosphetan-2,4-disulfid werden in 15 ml Toluol suspendiert und zwei Stunden unter Rückfluß erhitzt. Anschließend wird im Vakuum eingeengt und über eine Kieselgelsäule mit Methylenchlorid und steigenden Anteilen von Methanol gereinigt.

Ausbeute: 0,99 g (56 % der Theorie),

| | | | | | | | | |
|---|---|---|---|---|---|---|---|---|
| Ber.: | C | 66,06 | H | 6,65 | N | 6,16 | S | 14,08 |
| Gef.: | | 65,85 | | 6,58 | | 6,28 | | 14,26 |

$R_f$-Wert: 0,49 (Kieselgel, Methylenchlorid).

Beispiel I

Tabletten zu 25 mg 2-[N-Methyl-N-(2-(thienyl-3)-ethyl)-3-amino-propyl]-5,6-dimethoxy-1,3-dihydro-1-oxo-isoindol-hydrochlorid

Zusammensetzung:

1 Tablette enthält:

| | |
|---|---|
| Wirksubstanz | 25,0 mg |
| Maisstärke | 57,0 mg |
| Milchzucker | 48,0 mg |
| Polyvinylpyrrolidon | 4,0 mg |
| Magnesiumstearat | 1,0 mg |
| | 135,0 mg |

Herstellungsverfahren

Der Wirkstoff, Maisstärke, Milchzucker und Polyvinylpyrrolidon werden gemischt und mit Wasser befeuchtet. Die feuchte Mischung wird durch ein Sieb mit 1,5 mm-Maschenweite gedrückt und bei ca. 45°C getrocknet. Das trockene Granulat wird durch ein Sieb mit 1,0 mm-Maschenweite geschlagen und mit Magnesiumstearat vermischt. Die fertige Mischung preßt man auf einer Tablettenpresse mit Stempeln von 7 mm Durchmesser, die mit einer Teilkerbe versehen sind, zu Tabletten.
Tablettengewicht: 135 mg

Beispiel II

Dragées zu 20 mg 2-[N-Methyl-N-(2-(thienyl-3)-ethyl)-3-amino-propyl]-5,6-dimethoxy-1,3-dihydro-1-oxo-isoindol-hydrochlorid

1 Drageekern enthält:

| | |
|---|---|
| Wirksubstanz | 20,0 mg |

| | |
|---|---|
| Maisstärke | 41,5 mg |
| Milchzucker | 30,0 mg |
| Polyvinylpyrrolidon | 3,0 mg |
| Magnesiumstearat | 0,5 mg |
| | 95,0 mg |

Herstellungsverfahren

Der Wirkstoff, Maisstärke, Milchzucker und Polyvinylpyrrolidon werden gut gemischt und mit Wasser befeuchtet. Die feuchte Masse drückt man durch ein Sieb mit 1 mm-Maschenweite, trocknet bei ca. 45°C und schlägt das Granulat anschliessend durch dasselbe Sieb. Nach dem Zumischen von Magnesiumstearat werden auf einer Tablettiermaschine gewölbte Drageekerne mit einem Durchmesser von 6 mm gepreßt. Die so hergestellten Drageekerne werden auf bekannte Weise mit einer Schicht überzogen, die im wesentlichen aus Zucker und Talkum besteht. Die fertigen Dragees werden mit Wachs poliert.

Drageegewicht: 145 mg

Beispiel III

Ampullen zu 50 mg 2-[N-Methyl-N-(2-(thienyl-3)-ethyl)-3-amino-propyl]-5,6-dimethoxy-1,3-dihydro-1-oxo-isoindol-hydrochlorid

1 Ampulle enthält:

| | | |
|---|---|---|
| Wirksubstanz | | 50,0 mg |
| Sorbit | | 30,0 mg |
| Wasser für Injektionszwecke | ad | 2,0 ml |

Herstellungsverfahren

In einem geeigneten Ansatzgefäß wird der Wirkstoff in Wasser für Injektionszwecke gelöst und die Lösung mit Sorbit isotonisch gestellt.

Nach Filtration über einem Membranfilter wird die Lösung unter $N_2$-Begasung in gereinigte und sterilisierte Ampullen abgefüllt und 20 Minuten im strömenden Wasserdampf autoklaviert.

Beispiel IV

Suppositorien zu 30 mg 2-[N-Methyl-N-(2-(thienyl-3)-ethyl)-3-amino-propyl]-5,6-dimethoxy-1,3-dihydro-1-oxo-isoindol-hydrochlorid

---

1 Zäpfchen enthält:

| | |
|---|---|
| Wirksubstanz | 0,030 g |
| Hartfett (z.B. Witepsol H 19 und W 45) | 1,670 g |
| | 1,700 g |

Herstellungsverfahren:

Das Hartfett wird geschmolzen. Bei 38°C wird die gemahlene Wirksubstanz in der Schmelze homogen dispergiert. Es wird auf 35°C abgekühlt und in schwach vorgekühlte Suppositorienformen ausgegossen.

Beispiel V

Tropfenlösung mit 20 mg 2-[N-Methyl-N-(2-(thienyl-3)-ethyl)-3-amino-propyl]-5,6-dimethoxy-1,3-dihydro-1-oxo-isoindol-hydrochlorid

---

100 ml Lösungen enthalten:

| | |
|---|---|
| Wirksubstanz | 0,4 g |
| Hydroxyäthylcellulose | 0,15 g |
| Weinsäure | 0,1 g |
| Sorbitlösung 70 % Trockensubstanz | 30,0 g |

| | | |
|---|---|---|
| Glycerin | | 10,0 g |
| Benzoesäure | | 0,15 g |
| Dest.Wasser | ad | 100 ml |

Herstellungsverfahren:

Dest.Wasser wird auf 70°C erhitzt. Hierin wird unter Rühren Hydroxyäthylcellulose, Benzoesäure und Weinsäure gelöst. Es wird auf Raumtemperatur abgekühlt und hierbei das Glycerin und die Sorbitlösung unter Rühren zugegeben. Bei Raumtemperatur wird der Wirkstoff zugegeben und bis zur völligen Auflösung gerührt. Anschließend wird zur Entlüftung des Saftes unter Rühren evakuiert.

## Patentansprüche

1. Neue heteroaromatische Aminderivate der allgemeinen Formel

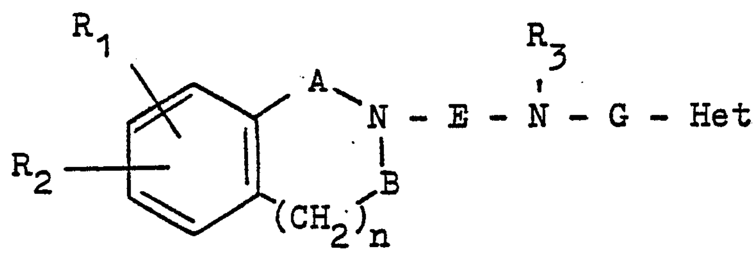 ,(I)

in der

n die Zahl 0 oder 1,

A eine Methylen-, Carbonyl- oder Thiocarbonylgruppe,

B eine Methylen-, Carbonyl- oder Thiocarbonylgruppe, wobei nur einer der Reste A oder B eine Thiocarbonylgruppe darstellen kann und in diesem Falle der andere der Reste A oder B eine Methylengruppe darstellen muß,

E eine gegebenenfalls durch eine Alkylgruppe mit 1 bis 3 Kohlenstoffatomen substituierte geradkettige Alkylengruppe mit 2 bis 4 Kohlenstoffatomen,

G eine gegebenenfalls durch eine Alkylgruppe mit 1 bis 3 Kohlenstoffatomen substituierte geradkettige Alkylengruppe mit 1 bis 6 Kohlenstoffatomen,

$R_1$ und $R_2$, die gleich oder verschieden sein können, Wasserstoffatome, Alkyl- oder Alkoxygruppen mit jeweils 1 bis 3 Kohlenstoffatomen in jedem Alkylteil oder $R_1$ und $R_2$ zusammen eine Alkylendioxygruppe mit 1 oder 2 Kohlenstoffatomen,

$R_3$ ein Wasserstoffatom, eine Alkenylgruppe mit 3 bis 5 Kohlenstoffatomen, eine Alkyl- oder Phenylalkylgruppe, wobei der Alkylteil jeweils 1 bis 3 Kohlenstoffatome enthalten kann, und

Het einen über ein Kohlenstoff- oder Stickstoffatom gebundenen 5- oder 6-gliedrigen heteroaromatischen Ring, welcher ein Sauerstoff-, Schwefel- oder Stickstoffatom, zwei Stickstoffatome oder ein Stickstoffatom und ein Sauerstoff- oder Schwefelatom enthält, an den zusätzlich über zwei benachbarte Kohlenstoffatome eine 1,3-Propylen-, 1,4-Butylen- oder 1,4-Buta-1,3-dienylengruppe gebunden sein kann, wobei in diesem Falle auch die Bindung über den Kohlenstoffring erfolgen kann, und in denen das Kohlenstoffgerüst der vorstehend erwähnten aromatischen und heteroaromatischen Ringe durch ein Halogenatom, eine Alkyl-, Hydroxy-, Alkoxy-, Phenylalkoxy-, Phenyl-, Dimethoxyphenyl-, Nitro-, Amino-, Acetylamino-, Carbamoylamino-, N-Alkyl-carbamoylamino-, Hydroxymethyl-, Mercapto-, Alkylmercapto-, Alkylsulfinyl-, Alkylsulfonyl-, Alkylsulfonyloxy-, Alkylsulfonylamino-, Alkoxycarbonylmethoxy-, Carboxymethoxy- oder Alkoxymethylgruppe mono- oder disubstituiert oder durch eine Methylendioxy- oder Ethylendioxygruppe substituiert sein kann und gleichzeitig eine gegebenenfalls vorhandene Iminogruppe in den vorstehend erwähnten heteroaromatischen Resten durch eine Alkyl-, Phenylalkyl- oder Phenylgruppe substituiert sein kann, wobei die vorstehend erwähnten Alkylteile jeweils 1 bis 3 Kohlenstoffatome enthalten können, bedeuten, deren N-Oxide, deren Enantiomere, deren Diastereomere und deren Säureadditionssalze.

2. Neue heteroaromatische Aminderivate der allgemeinen Formel

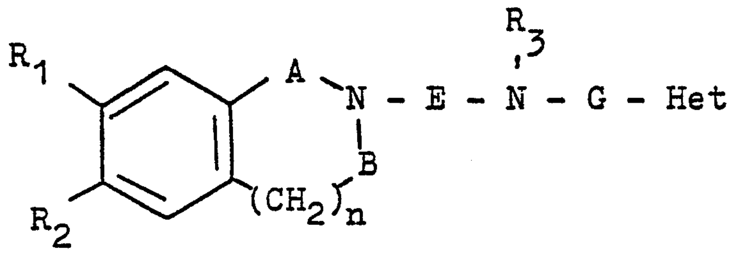

,(Ia)

in der

n die Zahl 0 oder 1,

A eine Methylen- oder Carbonylgruppe oder auch, wenn B eine Methylengruppe darstellt, eine Thiocarbonylgruppe,

B eine Methylen- oder Carbonylgruppe,

E eine n-Propylengruppe,

G eine Methylen-, Äthylen-, n-Propylen-, n-Butylen- oder n-Pentylengruppe,

$R_1$ und $R_2$, die gleich oder verschieden sein können, Wasserstoffatome, Methyl- oder Methoxygruppen oder $R_1$ und $R_2$ zusammen eine Methylendioxygruppe,

$R_3$ ein Wasserstoffatom, eine Methyl- oder Allylgruppe und

Het eine Pyrrolyl-2-, Pyrrolyl-3-, N-Methyl-pyrrolyl-2-, N-Methyl-pyrrolyl-3-, Furyl-2-, Furyl-3-, Benzo[b]furyl-2-, Benzo[b]furyl-3-, 7-Methyl-benzo[b]furyl-3-, 6-Methoxy-benzo-[b]furyl-3-, 5-Methoxy-3-phenyl-benzo[b]furyl-2-, Thienyl-2-, Thienyl-3-, 5-Methyl-thienyl-2-, 2,5-Dimethyl-thienyl-3-, 5-Brom-thienyl-2-, Benzo[b]thienyl-2-, Benzo[b]thienyl-3-,

6-Hydroxy-benzo[b]thienyl-3-, 6-Methoxy-benzo[b]thienyl-3-, 5,6-Dimethoxy-benzo[b]thienyl-3-, 2,5-Dimethyl-benzo[b]-thienyl-3-, 5-Methoxy-benzo[b]thienyl-2-, 6-Methoxy-benzo-[b]thienyl-2-, 6-Methylmercapto-benzo[b]thienyl-3-, 6-Methylsulfinyl-benzo[b]thienyl-3-, 6-Methylsulfonyl-benzo[b]-thienyl-3-, 6-Methylsulfonyloxy-benzo[b]thienyl-3-, 6-Ethoxycarbonylmethoxy-benzo[b]thienyl-3-, 6-Carboxymethoxy-benzo[b]thienyl-3-, 6-Dimethylamino-benzo[b]thienyl-3-, 6-Methylsulfonylamino-benzo[b]thienyl-3-, 6-Acetamino-benzo-[b]thienyl-3-, Benzo[b]thienyl-4-, Pyrazolyl-1-, Pyrazolyl-3-, 1,5-Dimethyl-pyrazolyl-3-, 1-Methyl-imidazolyl-4-, 2-(3,4-Dimethoxy-phenyl)-imidazolyl-4(5)-, Benzo[d]imidazolyl-1-, 2-Benzyl-benzo[d]imidazolyl-1-, Oxazolyl-4-, Oxazolyl-5-, Isoxazolyl-3-, 3-Methyl-isoxazolyl-5-, 4-Methyl-thiazolyl-5-, Pyridyl-2-, Pyridyl-3-, Pyridyl-4-, Pyridyl-3-N-oxid-, 4-Nitro-pyridyl-2-, 4-Amino-pyridyl-2-, 4-Acetylamino-pyridyl-2-, 4-Carbamoylamino-pyridyl-2-, 4-N-Methylcarbamoylamino-pyridyl-2-, Indolyl-2-, Indolyl-3-, 5-Methyl-indolyl-3-, 5-Methoxy-indolyl-3-, N-Methyl-indolyl-3-, 6,7-Dimethoxy-chinolyl-4-, 6,7-Dimethoxy-isochinolyl-4- oder 6,7-Dimethoxy-isochinolyl-4-N-oxid-Gruppe bedeuten, deren Enantiomere, deren Diastereomere und deren Säureadditionssalze.

3. Neue heteroaromatische Aminderivate der allgemeinen Formel Ia gemäß Anspruch 2, in der

A, B, E, G und n wie im Anspruch 2 definiert sind,

$R_1$ und $R_2$, die gleich oder verschieden sein können, Methyl- oder Methoxygruppen oder $R_1$ und $R_2$ eine Methylendioxygruppe,

$R_3$ ein Wasserstoffatom oder eine Methylgruppe und

Het eine Thienyl-, Furyl-, Pyridyl-, Pyridyl-3-N-oxid-, Benzo[b]furyl-2-, Benzo[b]furyl-3-, Benzo[b]thienyl-3-,

Benzo[b]thienyl-2-, Benzo[b]thienyl-3-, Indolyl-3- oder 6,7-Dimethoxy-isochinolyl-4-gruppe bedeuten, deren Enantiomere, deren Diastereomere und deren Säureadditionssalze.

4. 2-[N-Methyl-N-(2-(thienyl-3)-ethyl)-3-amino-propyl]-5,6-dimethoxy-1,3-dihydro-1-oxo-isoindol und dessen Säureadditionssalze.

5. 2-[N-Methyl-N-(2-(benzo[b]thienyl-3)-ethyl)-3-amino-propyl]-5,6-dimethoxy-1,3-dihydro-1-oxo-isoindol und dessen Säureadditionssalze.

6. Physiologisch verträgliche Säureadditionssalze der Verbindungen gemäß den Ansprüchen 1 bis 5 mit anorganischen oder organischen Säuren.

7. Arzneimittel, enthaltend eine Verbindung der allgemeinen Formel I gemäß den Ansprüchen 1 bis 5 oder dessen physiologisch verträgliches Säureadditionssalz gemäß Anspruch 6 neben einem oder mehreren inerten Trägerstoffen und/oder Verdünnungsmitteln.

8. Arzneimittel gemäß Anspruch 7 geeignet zur Behandlung der Herzinsuffizienz und von ischämischen Herzerkrankungen.

9. Verfahren zur Herstellung eines Arzneimittels gemäß den Ansprüchen 7 und 8, dadurch gekennzeichnet, daß auf nichtchemischem Wege eine Verbindung gemäß den Ansprüchen 1 bis 5 oder dessen physiologisch verträgliches Säureadditionssalz gemäß Anspruch 6 in einen oder mehrere inerte Trägerstoffe und/oder Verdünnungsmittel eingearbeitet wird.

10. Verfahren zur Herstellung von neuen heteroaromatischen Aminderivaten gemäß den Ansprüchen 1 bis 6, dadurch gekennzeichnet, daß

a) eine Verbindung der allgemeinen Formel

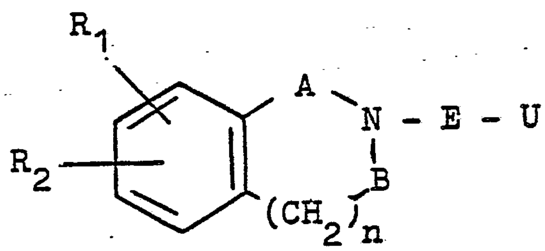

,(II)

mit einer Verbindung der allgemeinen Formel

V - G - Het ,(III)

in denen $R_1$, $R_2$, A, B, E, G, n und Het wie in den Ansprüchen 1 bis 5 definiert sind, wobei jedoch A oder B keine Thiocarbonylgruppe darstellen kann, einer der Reste U oder V die $R_3$'-NH-Gruppe, wobei $R_3$' eine Schutzgruppe für eine Aminogruppe darstellt oder die für $R_3$ in den Ansprüchen 1 bis 5 erwähnten Bedeutungen besitzt; und der andere der Reste U oder V eine nukleophile Austrittsgruppe darstellt, umgesetzt und gegebenenfalls anschließend ein verwendeter Schutzrest abgespalten wird oder

b) eine Verbindung der allgemeinen Formel

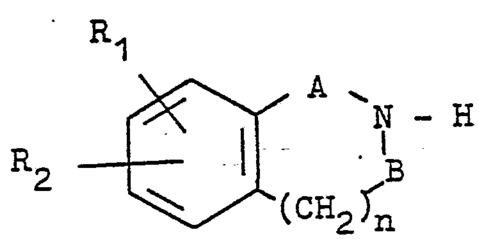

,(IV)

in der $R_1$, $R_2$, A, B und n wie in den Ansprüchen 1 bis 5 definiert sind, wobei jedoch A oder B keine Thiocarbonylgruppe darstellen kann, mit einer Verbindung der allgemeinen Formel

$$\begin{array}{c} R_3' \\ | \\ Z_1 - E - N - G - Het \end{array} \quad ,(V)$$

in der
E, G und Het wie in den Ansprüchen 1 bis 5 definiert sind,

$R_3'$ eine Schutzgruppe für eine Aminogruppe darstellt oder die für $R_3$ in den Ansprüchen 1 bis 5 erwähnten Bedeutungen besitzt und

$Z_1$ eine nukleophile Austrittsgruppe darstellt, umgesetzt und gegebenenfalls anschließend ein verwendeter Schutzrest abgespalten wird oder

c) eine Verbindung der allgemeinen Formel

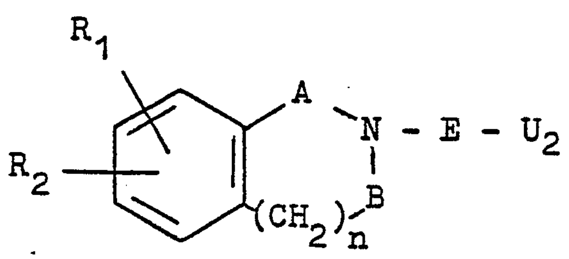

,(VI)

in Gegenwart einer Verbindung der allgemeinen Formel

$$V_2 - G - Het \quad ,(VII)$$

in denen
$R_1$, $R_2$, A, B, E, G, Het und n wie in den Ansprüchen 1 bis 5 definiert sind,

einer der Reste $U_2$ oder $V_2$ eine $R_3$-NH-Gruppe, wobei $R_3$ wie in den Ansprüchen 1 bis 5 definiert ist, und der andere der Reste $U_2$ oder $V_2$ zusammen mit einem Wasserstoffatom des benachbarten Kohlenstoffatoms des Restes G oder E, wobei E und G jeweils wie in den Ansprüchen 1 bis 5 definiert sind, ein Sauerstoffatom bedeuten, reduktiv aminiert wird oder

d) ein Säureamid der allgemeinen Formel

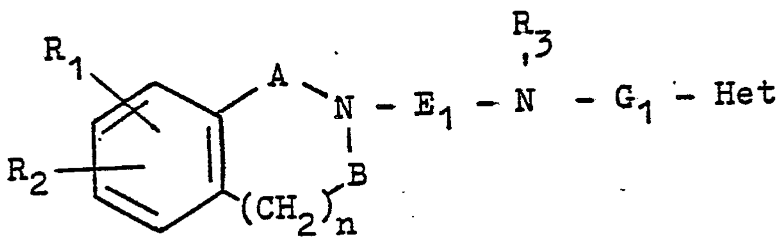

,(VIII)

in der $R_1$ bis $R_3$, A, B, Het und n wie in den Ansprüchen 1 bis 5 definiert sind, einer der Reste $E_1$ oder $G_1$ die für E oder G in den Ansprüchen 1 bis 5 erwähnten Bedeutungen aufweist und der andere der Reste $E_1$ oder $G_1$ ebenfalls die für E oder G in den Ansprüchen 1 bis 5 erwähnten Bedeutungen besitzt, wobei jedoch eine zu einem Stickstoffatom benachbarte Methylengruppe durch eine Carbonylgruppe ersetzt sein muß, reduziert wird oder

e) zur Herstellung von Verbindungen der allgemeinen Formel I, in der A oder B eine Thiocarbonylgruppe darstellt, eine Verbindung der allgemeinen Formel

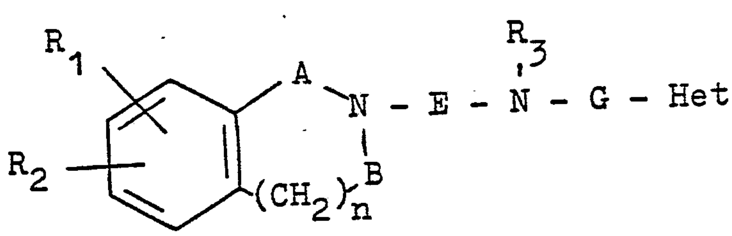

,(IX)

in der

$R_1$ bis $R_3$, A, B, E, G, Het und n wie in den Ansprüchen 1 bis 5 definiert sind, wobei jedoch einer der Reste A oder B eine Carbonylgruppe und der andere der Reste eine Methylengruppe darstellen muß, mit einem schwefeleinführenden Mittel umgesetzt wird oder

f) zur Herstellung von Verbindungen der allgemeinen Formel I, in der A und B jeweils eine $-CH_2$-Gruppe darstellen, eine Verbindung der allgemeinen Formel

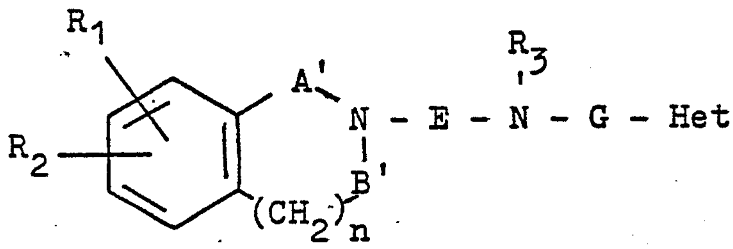 (X)

in der

$R_1$ bis $R_3$, E, G, Het und n wie in den Ansprüchen 1 bis 5 definiert sind,

einer der Reste A' oder B' eine Carbonyl- oder Thiocarbonylgruppe und

der andere der Reste A' oder B' eine Methylen-, Carbonyl- oder Thionylcarbonylgruppe darstellen, reduziert wird oder

g) zur Herstellung von Verbindungen der allgemeinen Formel I, in der A eine Methylengruppe darstellt, eine Verbindung der allgemeinen Formel

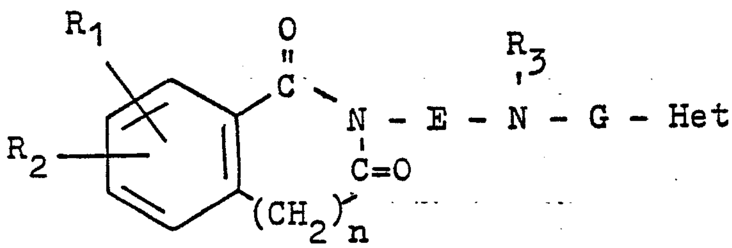 (XI)

in der

$R_1$ bis $R_3$, E, G, Het und n wie in den Ansprüchen 1 bis 5 definiert sind, reduziert wird

und gewünschtenfalls anschließend eine so erhaltene Verbindung der allgemeinen Formel I, welche mindestens ein chirales Zentrum enthält, in ihre Diastereomeren oder in ihre Enantiomeren aufgetrennt wird und/oder

eine so erhaltene Verbindung der allgemeinen Formel I in ihre Säureadditionssalze, insbesondere in ihre physiologisch verträglichen Säureadditionssalze mit anorganischen oder organischen Säuren übergeführt wird.